(19)

**Europäisches Patentamt**
**European Patent Office**
**Office européen des brevets**

(11)  **EP 3 463 669 B1**

(12)  **EUROPEAN PATENT SPECIFICATION**

(45) Date of publication and mention
of the grant of the patent:
**13.05.2020  Bulletin 2020/20**

(51) Int Cl.:
***B01L 7/02*** *(2006.01)*       ***B01L 7/00*** *(2006.01)*
***B01L 9/06*** *(2006.01)*       ***C12Q 1/68*** *(2018.01)*

(21) Application number: **17735657.3**

(86) International application number:
**PCT/SG2017/050293**

(22) Date of filing: **09.06.2017**

(87) International publication number:
**WO 2017/213592 (14.12.2017 Gazette 2017/50)**

(54) **RAPID THERMAL CYCLING FOR SAMPLE ANALYSES AND PROCESSING**

SCHNELLE THERMISCHE WECHSELBEANSPRUCHUNG ZUR ANALYSE UND VERARBEITUNG VON PROBEN

CYCLAGE THERMIQUE RAPIDE POUR ANALYSES ET TRAITEMENT D'ÉCHANTILLONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.06.2016  US 201662348155 P**
            **12.01.2017  SG 10201700260X**

(43) Date of publication of application:
**10.04.2019  Bulletin 2019/15**

(73) Proprietor: **STAR ARRAY PTE LTD**
**Singapore 618305 (SG)**

(72) Inventors:
- **GONG, Haiqing**
  **Singapore 589649 (SG)**
- **WEN, Yan**
  **Singapore 610347 (SG)**
- **ZENG, Xudong**
  **Singapore 640903 (SG)**

(74) Representative: **Berkkam, Ayfer**
**Berkkam Patent Consulting**
**Büklüm Sokak, No. 5/16-3**
**06680 Kavaklidere Ankara (TR)**

(56) References cited:
**WO-A1-2010/030647**       **WO-A2-2013/177429**

**Description**

**FIELD OF THE INVENTION**

The present invention relates to a method and an apparatus for performing amplification reaction of nucleic acids in a sample.

**BACKGROUND OF THE INVENTION**

Polymerase chain reaction (PCR) is increasingly important to molecular biology, food safety and environmental monitoring. A large number of biological researchers use PCR in their work on nucleic acid analyses, due to its high sensitivity and specificity. The time cycle of a PCR is typically in the order of an hour, primarily due to a time-consuming PCR thermal cycling process that is adapted to heat and cool reactors containing the sample to different temperatures for DNA denaturation, annealing and extension. Typically, the thermal cycling apparatus and method employs moving the reactors between two heating baths whose temperatures are set at the target temperatures as required for nucleic acid amplification reactions. Researchers have been constantly striving to increase the speed of thermal cycling.

Thermoelectric cooler (TEC) or Peltier cooler is also used as the heating/cooling element. However, it provides a typical ramping rate of 1- 5 degree C/sec which is rather slow in changing the temperature of the reactor and disadvantageously increases the time of the thermal cycling.

As an attempt to increase the PCR speed by reducing thermal mass, microfabricated PCR reactor with embedded thin film heater and sensor was developed to achieve faster thermal cycling at a cooling rate of 74 degree Celsius/s and a heating rate of around 60-90 degree Celsius/s. However, such a wafer fabrication process for making the PCR device is extremely expensive and thus is impractical in meeting the requirement of low cost disposable applications in biological testing.

Hot and cold air alternately flushing the reactors in a closed chamber to achieve higher temperature ramping than the TEC-based thermal cycler has been described. However, from the heat transfer point of view, air has much lower thermal conductivity and heat capacity than liquid, hence the temperature ramping of the air cycler is slower than that with a liquid. The TEC needs a significant amount of time to heat and cool itself and the heat block above the TEC. Further there is also need to overcome the contact thermal resistance between the heat block and the reactors.

Alternating water flushing cyclers were also developed in which water of two different temperatures alternately flush the reactors to achieve PCR speed. However, such devices contain many pumps, valves and tubing connectors which increase the complexity of maintenance and lower the reliability while dealing with high temperature and high pressure. With circulating liquid bath medium, the liquid commonly spills out from the baths.

Traditional water bath PCR cyclers utilize the high thermal conductivity and heat capacity of water to achieve efficient temperature heating and cooling. But, such cyclers have large heating baths containing a large volume of water which is hard to manage in loading and disposal, and also makes the heating time to target temperatures too long before thermal cycling can start. Such cyclers also have large device weight and high power consumption. The water tends to vaporize with usage and needs to be topped up. Besides, during the thermal cycling every time the reactor is alternately inserted into the baths, a layer of water remains adhered on the reactor body when taken out of each bath, thereby causing the change in temperature inside the reactor to get slower undesirably.

Researchers also tested moving heated rollers of different temperatures to alternately contact the reactors. However, use of long tubing reactors make it not only cumbersome to install and operate a large array of reactors, but also expensive. When the reactors are in a large array or a panel, it may be challenging to achieve heating uniformity among all the reactors.

The present invention provides an improved method and apparatus for enabling thermal cycling nucleic acid at an ultra-fast speed at affordable cost without using complex and expensive components or consumables. The apparatus is robust, light weight, easy to use, needs a small amount of bath medium in the baths and can handle disposable reactors for the reaction material to avoid cross contamination from one reactor to the next. This invention provides a great positive impact on biological analysis. The patent documents WO 2013/177429 A2 published on November 28, 2013 and WO 2010/030647 A1 published on March 18, 2010 disclose art known in the field.

**SUMMARY OF THE INVENTION**

Unless specified otherwise, the term "comprising" and "comprise" and grammatical variants thereof, are intended to represent "open" or "inclusive" language such that they include recited elements but also permit inclusion of additional, unrecited elements. The terminologies 'first bath', 'second bath' ..... 'sixth bath' do not constitute the corresponding number of baths in a sequence but merely are names for ease of identification with respect to the purpose they serve. These baths may not represent separate physical entities as some of them may be sharable. The term 'thermal processing' includes: a) thermal cycling, and optionally includes: b) thermal process steps before and/or after thermal cycling. The term 'thermal profile' refers to the temperature-time variation of the reactor(s) during a) alone or during a) with b).

The apparatus according to the invention is fully described in claim 1, while the method according to the invention is defined in claim 7. The following disclosure describes aspects of and related to the invention for illustrative purposes.

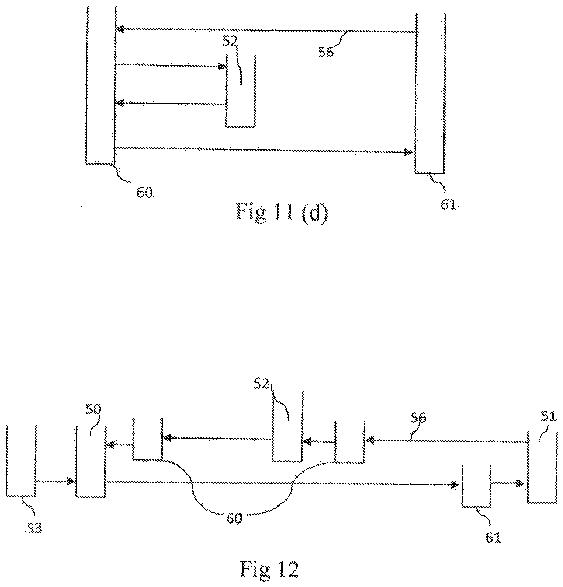

According to a first aspect, an apparatus is provided for thermal processing of nucleic acid in a thermal profile, the apparatus employing a reactor holder for holding reactor(s) each accommodating reaction material containing the nucleic acid and the reactor(s) being in any form such as tube(s) or wellplate(s) or chip(s) or cartridge(s), the apparatus comprising: a first bath; and a second bath, bath media in the baths being respectively maintainable at two different temperatures $T_{HIGH}$ and $T_{LOW}$; and a transfer means for allowing the reactor(s) to be in the two baths in a plurality of thermal cycles to alternately attain: a predetermined high target temperature $T_{HT}$, and a predetermined low target temperature $T_{LT}$, while the apparatus adapts to a temperature-offset feature defined by at least one condition from the group consisting: a) the $T_{HT}$ is lower than the $T_{HIGH}$, b) the $T_{LT}$ is higher than the $T_{LOW}$, and c) the conditions at a) and b), the transfer means being operable by at least one mode from the group consisting: a temperature guided motion controlling means (TeGMCM) that is operable based on the real-time temperature as sensed by a reactor temperature sensor during thermal cycling, and a time guided motion controlling means (TiGMCM) that is operable based on the time-periods for which the reactor(s) are allowed to be in the baths, the bath medium in any of the baths being in any phase including air, liquid, solid, powder and a mixture of any of these. Advantageously herein the concept of Newton's law is made use of which states that the rate of heat loss of a body is proportional to the difference in temperatures between the body and its surroundings. By maintaining the first bath temperature at $T_{HIGH}$ that is significantly higher than the $T_{HT}$ and the second bath temperature at $T_{LOW}$ that is significantly lower than the $T_{LT}$, the reactor(s) can undergo thermal cycling significantly faster. The TiGMCM can be user calibrated for the time-periods. TeGMCM allows better automation and accuracy but requires very fast temperature sampling and signal processing electronics, fast data communication with the reactor transfer mechanism, and very responsive mechanical motion components such as motors and actuators in the reactor transfer mechanism. TiGMCM on the other hand does not require such highly responsive set-up though needs user calibration based on the extent of the temperature offset. The advantageous impact of the temperature-offset feature has been demonstrated by experimental graphs at Figs. 12(a) and (b) where the time of thermal cycling with forty cycles has been shown to reduce to one-fifth. This reduction in time can be further reduced by increasing the magnitude of the temperature-offsets.

According to an embodiment, a third bath is provided where the bath medium is maintainable at a medium temperature $T_{MEDIUM}$ for thermal cycling the reactor(s) in three-steps, where the transfer means allows the reactor(s) to be in the third bath to attain a predetermined medium target temperature $T_{MT}$. The $T_{MT}$ may be lower than the $T_{MEDIUM}$ for attaining a faster heating rate from the $T_{LT}$ or the $T_{MT}$ may be higher than the $T_{MEDIUM}$ for attaining a faster cooling rate from the $T_{HT}$. The $T_{MT}$ may be maintained same as the $T_{MEDIUM}$.

According to an embodiment, a fourth bath is provided where the bath medium is maintainable at a temperature $T_{AP}$ to allow an additional process for the reactor(s) before the thermal cycling, the additional process being one from the group consisting: reverse transcription-polymerase chain reaction (RT-PCR), hot start process and isothermal amplification reaction, where the transfer means allows the reactor(s) to attain an additional process target temperature $T_{APT}$, the $T_{AP}$ being same as or higher or lower than the $T_{APT}$. The advantage is same as in using the temperature-offset feature. This helps in integrating the process steps and advantageously allows bath sharing as well with the appropriate temperature setting, thereby saving on foot print and mass of the apparatus.

The bath medium in the first bath may be maintainable above 100 degrees Celsius, to better exploit the advantage of the temperature-offset feature with a suitable bath medium that can be heated to such high temperatures. The bath medium in the second bath may be maintainable below room temperature to better exploit the advantage of the temperature-offset.

According to the invention, the transfer means is calibrated to initiate lift-off of the reactor(s) from the bath(s) when the reactor(s) reach a first lift-off temperature that is lower than the $T_{HT}$ and a second lift-off temperature that is higher than the $T_{LT}$, in order to compensate for operational electro-mechanical delays that unwantedly cause over heating or over cooling of the reactor(s).

According to an embodiment the apparatus comprises altering means for altering temperature in any bath during thermal cycling. This feature significantly helps reduce the number of baths in the apparatus thereby reducing the foot print and weight of the apparatus.

The $T_{HT}$ may be in the region 85-99 degree Celsius for denaturation of the nucleic acid, and the $T_{LT}$ may be in the region 45-75 degree Celsius for annealing of primers or probes onto nucleic acid or for primer extension, the first and the second baths being for thermal cycling the reactor(s) to attain polymerase chain reaction (PCR) amplification or primer extension.

The apparatus may further comprise a fifth bath for a temperature stabilization step in the thermal profile. The temperature stabilization step may be at one of the target temperatures if required for the thermal profile.

The apparatus may further comprise fluorescence imaging means or electrochemical detection means for analyses of the nucleic acid when the reactor(s) is in any of the baths or in air outside the baths.

The apparatus may further comprise a sixth bath wherein the bath medium is liquid or hot air that is maintainable at 40-80 degree C, wherein the bath medium and at least a portion of the bath wall is transparent to allow transmission of illumination light from a light source and transmission of emitted light from the reactor(s).

A first offset between the $T_{HIGH}$ and the $T_{HT}$ may be in the range 1- 400 degree Celsius and a second offset between the $T_{LOW}$ and the $T_{LT}$ may be in the range 1- 100 degree Celsius. Higher the offset, faster is the change of temperature attained by the reactor(s), thereby increasing the speed of the thermal cycling.

The bath medium in the first bath may be a first liquid added to a second liquid with a higher boiling point such that the temperature in the mixture can be maintained at a higher value such as above than 100 deg Celsius without boiling off.

The apparatus may further comprise bath cover(s), the cover(s) opening to allow the reactor(s) to be in the bath(s) and closing after the reactor(s) is/are removed from the bath(s). This feature helps is saving energy by reducing the heat lost or gained when kept exposed to the ambience. Other parts of the apparatus are also prevented from getting heated up particularly when the $T_{HIGH}$ is set to a value much higher than the ambience temperature. This also reduces vaporization of the bath medium and contamination of the surrounding parts of the apparatus.

The apparatus comprises bath temperature sensors to monitor temperatures of the bath media and a reactor temperature sensor that is capable of moving with the reactor holder during thermal cycling, to monitor the real time temperature of the reactor(s). The apparatus may further comprise a vessel containing a substance to encapsulate the reactor temperature sensor, the vessel and the substance having similar construction or heat transfer characteristics to that of the reactor(s) and the reaction material so that the temperature sensed by the reactor temperature sensor is close to the temperature of the reaction material in the reactors at any instant.

The apparatus may further comprise a seventh bath that can receive the reactor(s) and be progressively heated while conducting melt curve analysis after the thermal cycling. This helps integrating the process steps and particularly advantageous in terms of bath sharing.

According to a second aspect, method claims corresponding to the apparatus claims are provided.

The present invention enables the entire process of thermal cycling of nucleic acid and analysis to be completed in a very short time duration of a few minutes, from bath heating preparation, to reactor thermal cycling and fluorescence signal acquisition. The invention provides scope for bath sharing.

## BRIEF DESCRIPTION OF THE DRAWINGS

In the following drawings, same reference numbers generally refer to the same parts throughout. The drawings are not to scale, instead the emphasis is on describing the concept.

Fig. 1a is a schematic view of an embodiment of a set up for thermal cycling of a reaction material containing nucleic acid as according to the invention.

Fig. 1b is a perspective view of the reactors being accommodated in an exemplary biochip that is usable for the invention.

Fig. 1c is a cross-sectional view to illustrate the illumination and fluorescence emission detection module with an opaque tubular reactor that is particularly suited for powder bath medium.

Fig. 2 is an isometric view of an embodiment of the apparatus for the process of thermal cycling as at Fig. 1.

Fig. 3 is an isometric view of an embodiment of the reactor array and baths with optics modules of the apparatus in Fig. 2.

Fig. 4a is an exemplary graphical representation of a typical 2-step thermal cycling process along with melt curve analysis, according to an embodiment.

Fig. 4b is an exemplary graphical representation of a typical 3-step thermal cycling process along with melt curve analysis, according to an embodiment.

Fig. 5a is graphical representation of Newton's Law for heating rate.

Fig. 5b is graphical representation of Newton's Law for cooling rate.

Fig. 6(a) is a graphical representation of experimental reactor temperature variations with time during forty cycles of thermal cycling using the apparatus previously described and without using the temperature offset feature.

Fig. 6(b) is a graphical representation of experimental reactor temperature variations with time during forty cycles of thermal cycling using the apparatus previously described and using the temperature offset feature.

Figs. 7(a) to (e) are graphical representations to describe the concept of temperature-offset feature according to an embodiment of the invention.

Figs. 8(a) and (b) are a schematic and elevational cross-sectional representations to describe the concept of temperature-offset feature according to various embodiments of the invention.

Fig. 8(c) is a graphical representation to describe the concept of temperature-offset feature according to Figs. 8(a) and (b).

Fig. 9(a) to (d) are graphical representations to describe the concept of temperature-offset feature.

Figs. 10 to 12 are schematic representations to describe the concept of temperature-offset feature according to various embodiments of the invention.

## DETAILED DESCRIPTION

The following description presents several preferred embodiments of the present invention in sufficient detail such that those skilled in the art can make and use the invention.

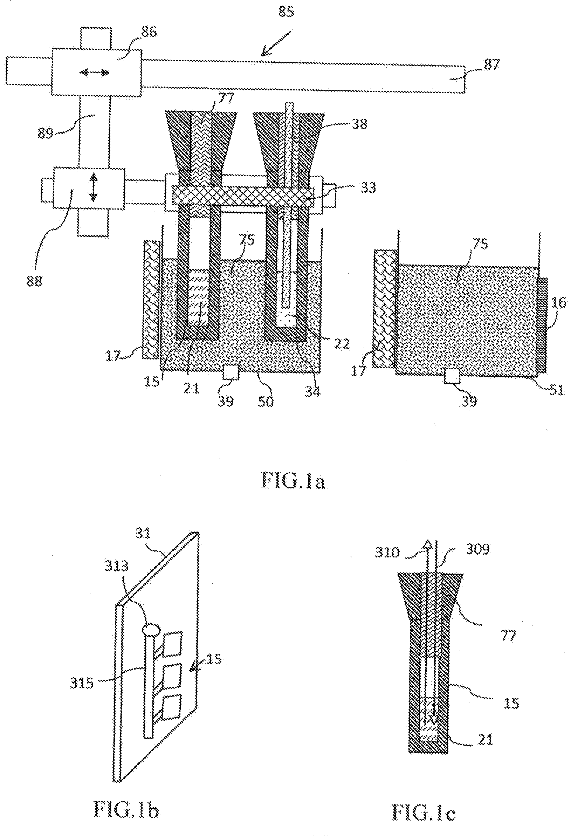

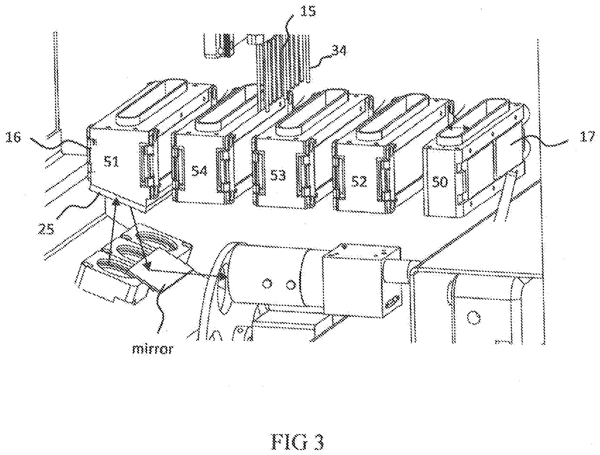

**Fig 1a** shows a schematic view of an embodiment of a portion of the thermal cycling apparatus for thermal cycling of nucleic acid such as for PCR, primer extension or other enzymatic reactions. The apparatus has two baths 50 and 51 containing the bath medium 75. Each bath 50 or 51 has a bath heater 17 and a bath temperature sensor 39 mounted along the bath surface to enable control of the temperature of the bath medium 75. According to another embodiment, the bath temperature sensors 39 may be positioned inside the baths 50, 51. Bath 50 may be suitable for the step of denaturation and the bath 51 may be suitable for the step of annealing and/or extension. The cooler 16 is useful when the bath 51 needs to be actively cooled to below room temperature. The bath medium 75 shown here is liquid, however any other type of fluid or powder or solid bath medium 75 may also be used. For some embodiments, the bath heater 17 on the low temperature bath 51 is optional, if bath 51 does not have to be heated. For the thermal cycling, the reactor 15 is alternately transferred between the baths 50, 51 multiple times. To enable fast plunging of the reactor 15 into the bath medium 75, slim reactor 15 is preferable such as glass capillaries. The reactor 15 is sealed with a sealant or a cap 77 and a portion of the reactor 15 herein is transparent to allow light to pass through for dye or probe excitation and fluorescence imaging. A temperature monitoring unit 34 is installed on the reactor holder 33 and moves along with the reactor 15 between the baths 50, 51. The temperature monitoring unit 34 contains a fast response temperature sensor 38 inside. The temperature monitoring unit 34 has a shape similar to that of the reactor 15 and is constructed to have a similar or the same steady state and transient thermal characteristics as those of the reactor 15, for the temperature reading and thermal response to be similar or same as those of the reactor 15 unless another reactor 15 itself is used for the purpose. For example, the temperature monitoring unit 34 may have the fast response temperature sensor 38 inserted into water or oil or a layer of oil over water 22 and sealed. Although only one reactor 15 is shown, according to other embodiments the reactor holder 33 may accommodate a plurality of reactors 15. The reactor 15 may be in the form of tube(s) as shown or as wellplate(s) or chip(s) or cartridge(s). The reactor transfer mechanism 85 transfers the reactor 15 and the temperature monitoring unit 34 at high speed among the baths 50 and 51 to expose them alternately to the different temperatures in the baths 50 and 51 as required for the thermal cycling. There are many possible designs of the reactor transfer mechanism 85. One such mechanism is comprised of an X stage 86 moving along an X axis linear guide 87 for the reactor 15 and the temperature monitoring unit 34 to reach to a region above the baths 50 and 51, and a Z stage 88 moving along a Z axis linear guide 89 for the reactors 15 to move them down to enter the bath medium 75 or to be withdrawn from the bath medium 75. Such a transfer mechanism 85 can also consist of a rotary arm (not shown) that moves the reactor 15 and the temperature monitoring unit 34 in an angular direction along with the Z stage 88 moving along the Z axis linear guide 89. The reactors 15 have an opening for loading and the reaction material 21 and the openings are sealable. The sealant 77 may be made of a silicone rubber or UV cured polymer, hot melt and/or wax and/or gel which is in solid phase during thermal cycling. The sealing can also be achieved using liquid such as oil, viscous polymer, and gel. The highly viscous liquid can be applied to the opening and/or top section of the reactors 15, it is able to block the vapor generated from the reaction material 21 from leaking out. The arrangement for the fluorescent imaging may be in any form as in the art. **Fig. 1b** is a perspective view of the reactors 15 being accommodated in a card 31 for use with the baths as according to an alternate embodiment for the reactors 15. There is at least one inlet 313 which is in fluid communication with the reactors 15 via a network of channels 315. The reaction material 21 to be tested can be loaded into the inlet 315 that subsequently flows into the reactors 15. **Fig 1c** shows an embodiment particularly where the reactor 15 is made of a non-transparent reactor 15. For the fluorescent imaging, an optical fiber 309 transmits light from an illumination light source such as an LED (not shown) into the reaction material 21 inside the reactor 15. The optical fiber 310 is for light transmission from the reaction material 21 to a photodetector (not shown). The sealant or cap 77 holds the optical fibers 309, 310. Alternately, imaging may also be conducted for at least partially transparent reactor 15 using at least partially transparent bath(s) with transparent bath medium 75 as shown in **Fig. 3**. In some cases imaging may be conducted when the reactor 15 is outside any bath. Fluorescence imaging of the reactors 15 while the reactors 15 are inside the bath medium 75 is important since the reactors 15 may have to be maintained at the annealing or extension temperature for a prolonged period of time when multiple images of different wavelengths are taken for multiplex detection or to acquire fluorescence images of control genes. At least a portion of the reactor 15 needs to be transparent to allow light to pass through for dye or probe excitation and fluorescence imaging.

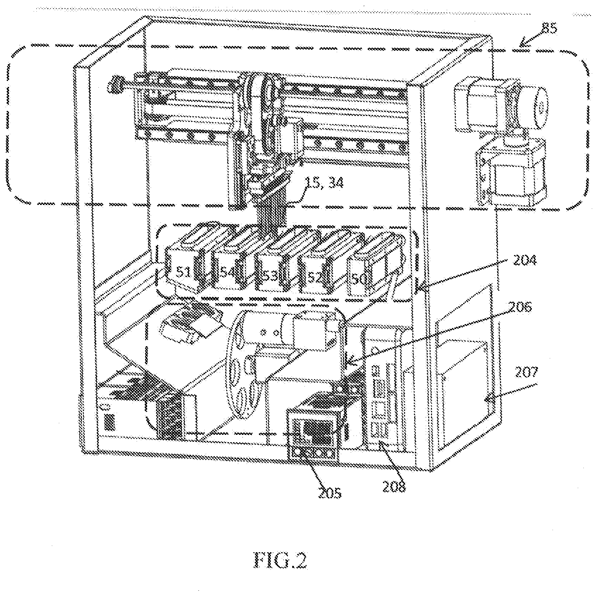

**Fig 2** shows an isometric view of the apparatus with the reactor transfer mechanism 85, wherein an optics module 206 carries out fluorescent detection of nucleic acid inside the reactors 15, a temperature controller module 205 controls the bath temperatures, a motion controller 207 controls all motions and a system controller 208 controls the system, with data communication and processing, image processing and data analysis. The bath module 204 is shown to comprise five baths 50, 51, 52, 53, 54 placed next to each other and each maintained at a predetermined temperature. In the optics module 206, the reactors 15 are situated in the low temperature bath 51 having air or transparent liquid as the bath medium 75 in this embodiment. The additional processes like reverse transcription-polymerase chain reaction (RT-PCR), hot start process, and isothermal amplification reaction can also be carried out in any of the baths for thermal cycling which can be set at temperature $T_{AP}$ before the thermal cycling and reset to the temp for thermal cycling after the completion of the above additional processes. **Fig 3** shows a blown up illustration of the bath module 204 and the optical module 206. The array of reactors 15 with the temperature monitoring unit 34 are transferred among the baths 50 to 54. In this embodiment, the bath 51 has air or transparent liquid as the bath medium 75 along with a transparent window 25 at the bottom to pass the illumination light and emission light to and from the reactor 15 as shown with the arrows for the ray paths. The bath heaters 17 or cooler 16 are connected to the sidewalls of the baths. The apparatus may may further comprise a hot air zone (not shown) for placing the reactors 15 particularly during imaging. This simplifies the apparatus. There may be an electrical heater or an infrared heater to form the hot air zone above a bath or inside a bath. The heater may preferably be installed with the reactor holder 33 so that only the air in the vicinity of the reactors 15 is heated while the reactor(s) 15 is/are moving between the baths. This feature saves energy and saves the other parts of the apparatus from getting undesirably heated. The multiple baths 50 to 54 may be used as required under the thermal profile for the thermal cycling and for steps before and after the thermal cycling. In a three-step thermal profile for thermal cycling, the reactor 15 is inserted into three baths within each thermal cycle. In between the baths 50 and 51, the reactor 15 with the monitoring unit 34 may be inserted into a 3rd bath at a medium temperature or positioned in hot air for a period of time required for annealing and or extension. Before the thermal cycling, the reactor 15 with the temperature monitoring unit 34 may be inserted into a four bath, that is maintained at a predetermined temperature such as for an additional processing before the thermal cycling for nucleic acid amplification. The additional process may be from the group consisting reverse transcription-polymerase chain reaction (RT-PCR), hot start process and isothermal amplification reaction. Melt curve analysis may be conducted using a bath after the thermal cycling.

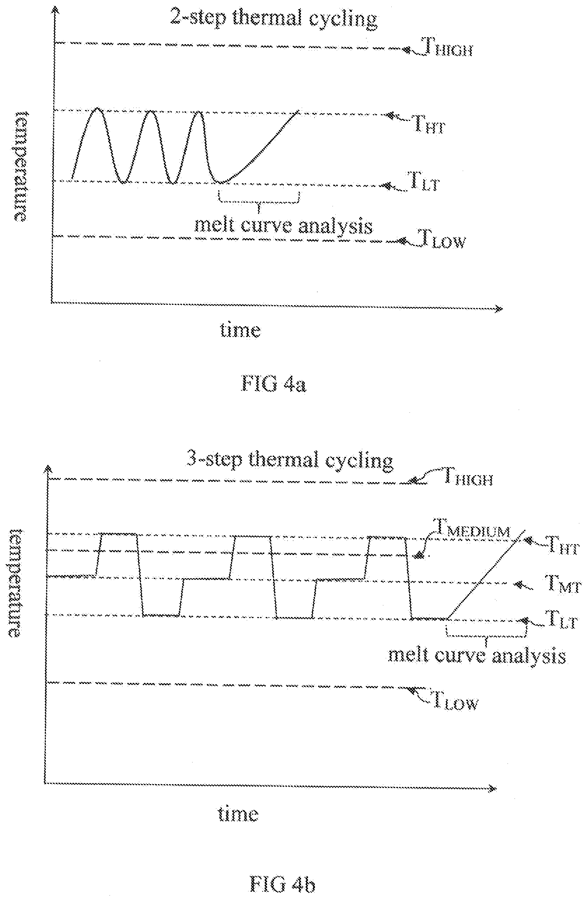

**Figs 4a** is an exemplary time-temperature graphical representations of typical 2-step thermal cycling process followed by a melt curve analysis. Only three cycles are shown over the processes of denaturation and annealing employing two baths. After the thermal cycling, the reactors 15 are placed in at least a partially transparent bath medium 75 that is progressively heated while melt curve analysis is conducted. The fluorescence signals from the reactors 15 are acquired at multiple temperatures to form a fluorescence-temperature curve for the melt curve analysis. The speed of thermal cycling is increased by increasing the ramp-up and ramp-down of the temperatures of the reactors 15 by the temperature-offset feature, where the temperature of the bath medium 75 in the first bath 50 is maintained at a temperature $T_{HIGH}$ which is significantly higher than the temperature for denaturation or the target high temperature $T_{HT}$ and the temperature of the bath medium 75 in the second bath 51 is maintained at a temperature $T_{LOW}$ which is significantly lower than the temperature for annealing or the target low temperature $T_{LT}$. This feature advantageously uses Newton's law of cooling that states: 'the rate of change of the temperature of an object is proportional to the difference between its own temperature and the ambient temperature (i.e. the temperature of its surroundings). Fig. 4b is an exemplary time-temperature graphical representations of typical 3-step thermal cycling process followed by a melt curve analysis. Only three cycles are shown over the processes of denaturation, annealing and extension employing three baths. After the thermal cycling, the melt curve analysis is conducted as described under Fig. 4a. Herein, the temperature-offset feature described under Fig. 4a is further extended for the third bath 52 where the temperature of the bath medium 75 is maintained at a temperature $T_{MEDIUM}$ which is significantly higher than the temperature for extension or $T_{MT}$. The bath medium 75 for the melt curve analysis may be air or transparent liquid and the bath needs to have a transparent window 25, both required to have low auto-fluorescence.

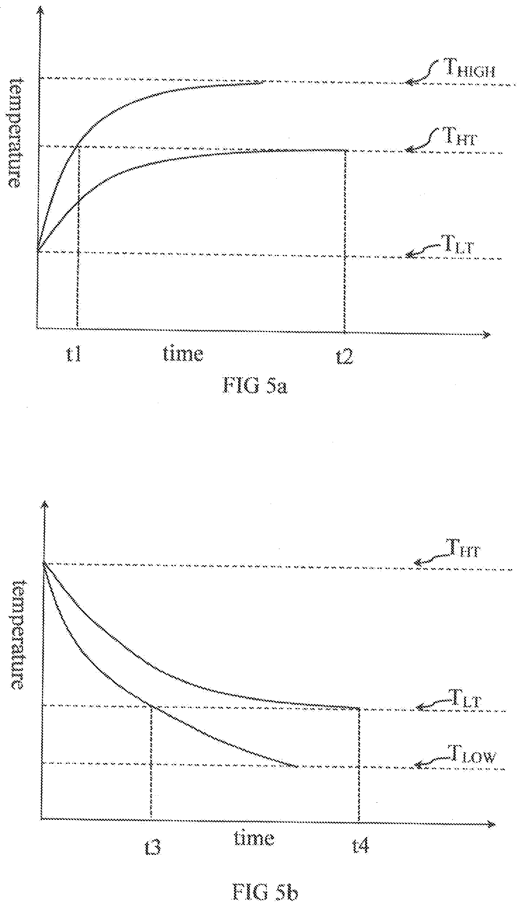

**Fig. 5a** illustrates this law, where an object at temperature $T_{LT}$ is shown to heat up to a temperature $T_{HT}$ at a time t1 when the ambient temperature is at a temperature $T_{HIGH}$, where $T_{HIGH} > T_{HT}$. The same object at temperature $T_{LT}$ is shown to heat up to the temperature $T_{HT}$ at a time t2 when the ambient temperature is at a temperature $T_{HT}$. As shown, t1<t2, which indicates that the rate of heating is faster when the ambient temperature is higher. **Fig. 5b** illustrates this law, where an object at temperature $T_{HT}$ is shown to cool down to a temperature $T_{LT}$ at a time t3 when the ambient temperature is at a temperature $T_{LOW}$, where $T_{LOW} < T_{LT}$. The same object at temperature $T_{HT}$ is shown to cool down to the temperature $T_{LT}$ at a time t4 when the ambient temperature is at a temperature $T_{LT}$. As shown, t3<t4, which indicates that the rate of cooling is faster when the ambient temperature is lower. Thus, by maintaining the $T_{HIGH}$ to be significantly higher than the temperature for denaturation and $T_{LOW}$ to be significantly lower than the temperature for annealing, the rate of change of the temperature of the reactor 15 is significantly increased during the thermal cycling, thereby making the thermal cycling significantly faster.

As described under Figs 4a and 4b, the concept of using the temperature-offset may be used at any stage of the thermal cycling using any number of baths as required. This feature of maintaining a temperature-offset may selectively be used for only heating or only cooling steps or for both. The liquid bath medium 75 in the high temperature bath 50 may have a boiling temperature that is higher than that of water at 100 degree Celsius to enable faster ramp-up of the reactor temperature.

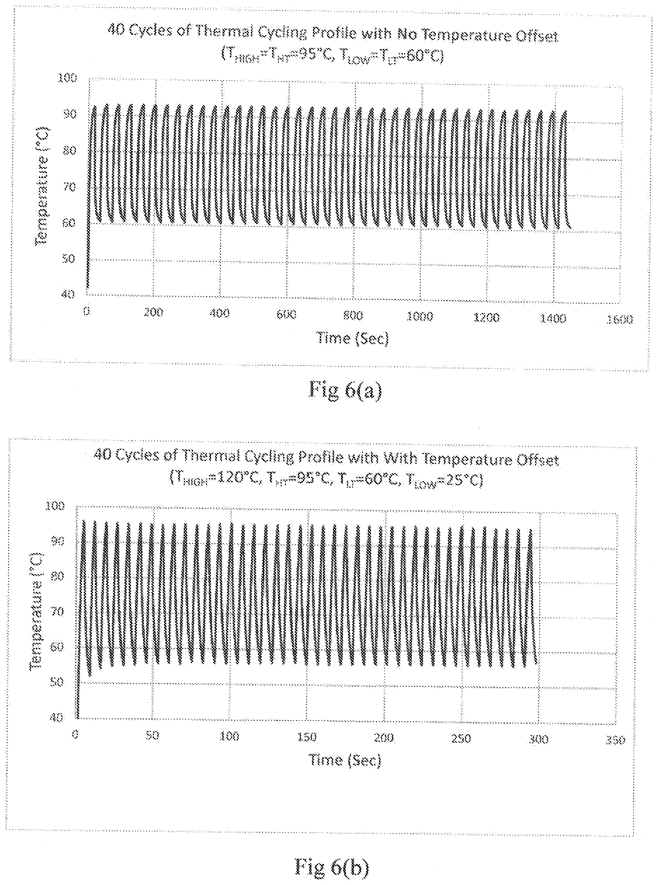

**Fig. 6(a)** shows a graphical representation of experimental reactor temperature variations with time during forty cycles of thermal cycling using the apparatus previously described and without using the temperature offset feature by maintaining $T_{HIGH} = T_{HT} = 95$ degree Celsius and $T_{LOW} = T_{LT} = 60$ degree Celsius. The time taken for forty thermal cycles is 1450 seconds as seen from the time axis. **Fig. 6(b)** shows a graphical representation of experimental reactor temperature variations with time during forty cycles of thermal cycling using the apparatus previously described and using the temperature offset feature by maintaining $T_{HIGH} = 120$ degree Celsius, $T_{HT} = 95$ degree Celsius, $T_{LOW} = 25$ degree Celsius and $T_{LT} = 60$ degree Celsius. The time taken for forty thermal cycles is 300 seconds as seen from the time axis. Thus, with the temperature-offset feature, the time for thermal cycling has been reduced to about one-fifth. This is a very significant improvement as about 25 minutes has been reduced to 5 minutes. In this case slight overshoots are observed in both the high and low temperature baths 50, 51 due to delay in the electromechanical system in changing the baths. Such overshoots can be avoided by calibrating the apparatus to lift-off the reactors 15 from the baths 50, 51 slightly before the target temperatures are attained to compensate for the delay.

To enable rapid heating of the reactor 15, according to an embodiment the liquid bath medium 75 in the high temperature bath 50 is significantly over-heated to 125 degree Celsius and above the high target temperature $T_{HT}$ for DNA denaturation which is typically around 95 degree Celsius. A mixture of glycerol and water of a mixing ratio of 70:30 or higher can be used to avoid the liquid boiling when above 100 degrees Celsius while maintaining a good thermal conductivity of the liquid. To enable rapid cooling of the reactor 15, the liquid in the low temperature bath 51 is significantly cooled below the low target temperature $T_{LT}$ for annealing and/or extension DNA molecules which is typically around 58 degree Celsius. For example, the liquid in the low temperature bath 51 may be maintained at 10 or 30 degrees Celsius while the room temperature is at 20°C. The reactors 15 contain a reaction material 21 having least one nucleic acid molecule and a reagent for analyses.

A temperature guided motion controlling means (TeGMCM) or a time guided motion controlling means (TiGMCM) (not shown) is employed in the apparatus for allowing the reactors 15 to remain in the baths 50 to 54 until substantially the corresponding target temperature is attained, irrespective of the temperatures of the corresponding baths. The TeGMCM/ TiGMCM may be fed with advance signals when the reactor(s) 15 are about to reach the target temperatures as sensed by the temperature monitoring unit 34 in order to avoid over heating or over cooling of the reactors 15 due to any operational electro-mechanical delay in removing the reactor 15 from the bath. This helps to maintainin better accuracy of the predetermined target temperatures attained by the reactors 15. Advantageously, with the TeGMCM/ TiGMCM, the required number of high temperature and low temperature baths can be reduced in the apparatus by allowing the reactor 15 to attain multiple levels of temperature based on the time for which it is allowed to remain in the bath. For stabilization at any temperarure level however dedicated bath at that temperature is required.

The above temperature-offsets with the over-heating and under-heating methods make the temperature ramp-up/ramp-down for the reactor (15) faster. The theory is explained in this section containing Eq (1-7). One type of our reactor 15 in operation comprises a tube containing the reaction material. The tube and the reaction material 21 have different thermal and other material properties. To illustrate the heat transfer characteristics of the reaction material 21 loaded reactor 15 submerged in bath medium 75 during thermal cycling, we approximate the submerged reactor 15 loaded with the reaction material 21 as a cylinder made of a homogeneous material having the outer surface area $A_s$, the radius R, the length L, the volume of the submerged cylinder V, the heat capacity $c_p$. In order to estimate the time of heating of the cylinder submerged in the high temperature bath from the $T_{LT}$ when the cylinder enters the high temperature bath to the $T_{HT}$ during thermal cycling, we determine the average rate of heat transfer $\dot{Q}_{avg}$ from Newton's Law of Cooling by using the average surface temperature $T_{s, avg}$ of the cylinder [Reference 1: Y.A. Cengel and A.J. Ghajar, Heat and Mass Transfer: Fundamentals and Applications, Fifth Edition In SI Units by McGraw-Hill Education, 2015]. That is,

$$\dot{Q}_{avg} = -hA_s(T_{s, avg} - T_{HIGH}), \qquad \dots\dots\dots \text{Eq (1)}$$

where $T_{HIGH}$ is the temperature of bath medium in the high temperature bath, $T_{s, avg} = (T_{LT} + T_{HT})/2$. Under the over-heating scheme in the high temperature bath, $T_{HIGH} > T_{HT}$. Note: an example of the $T_{LT}$ is the annealing temperature in PCR, and an example of the $T_{HT}$ is the denaturation temperature in PCR.

Next, we determine the total heat transferred from the cylinder [Reference 1], which is simply the change in energy of the cylinder as it heats from $T_{LT}$ to $T_{HT}$:

$$Q_{total} = \Box V c_p (T_{HT} - T_{LT}), \qquad \dots\dots\dots \text{Eq (2)}$$

In this calculation, we assumed that the entire cylinder is at uniform temperature over the domain of the cylinder. With this assumption, the time of heating the cylinder from $T_{LT}$ to $T_{HT}$, $\Delta t$, is determined to be

$$\Delta t = \frac{Q_{total}}{\dot{Q}_{avg}} = \frac{V c_p (T_{LT} - T_{HT})}{hA_s[0.5(T_{HT} + T_{LT}) - T_{HIGH}]} \qquad \dots\dots\dots \text{Eq (3)}$$

Because of the assumption made above, Eq (3) does not yield accurate temperature value, but it reveals the factors of influencing the time of heating up the cylinder.

The heat transfer coefficient h in Eq (3) is related to the relative velocity between the heating medium and the reactor, which can be obtained from the following analysis of convective heat transfer in an external flow across a cylinder [Reference 1]:

The average Nusselt number for flow across the cylinder can be expressed compactly as

$$Nu = CR_e^m P_r^n, \qquad \dots\dots\dots \text{Eq (7-37)}$$

of Reference 1

where the constant C, m, and n are related to the Reynolds number $R_e$ which is defined as $R_e = \dfrac{\rho v D}{\mu}$

where D is the diameter of the cylinder, $\rho$ is the density of the liquid in bath, $\mu$ is the dynamic viscosity of the liquid in bath, and v is the relative velocity between the heating medium and the reactor.

For example, if $R_e$ is in the range of 40 - 4,000, Eq (7-37) can be rewritten as

$$Nu = 0.683 R_e^{0.466} P_r^{1/3} \qquad \text{Eq (4)},$$

which is as shown in Table 7-1 in Reference 1.

Since $Nu = \dfrac{hD}{k}$, where h is the heat transfer coefficient on the cylinder surface, D is the diameter of the cylinder, and k is the thermal conductivity of liquid in the bath, Eq (4) can be rewritten as

$$h = 0.683 \frac{k}{D} \left(\frac{\rho D}{\mu}\right)^{0.466} P_r^{1/3} (v)^{0.466} \qquad \text{Eq (5)},$$

Inserting Eq (5) into Eq (3), we obtain

$$\Delta t = \frac{Q_{total}}{\dot{Q}_{avg}} = \frac{Vc_p\,(T_{HT} - T_{LT})}{A_s[T_{HIGH} - 0.5(T_{HT} + T_{LT})]0.683\frac{k}{D}\left(\frac{\rho D}{\mu}\right)^{0.466}P_r^{1/3}v^{0.466}} \quad \dots\dots\dots\dots\dots\dots\dots\dots Eq\ (6)$$

Eq (6) shows that advantage of implementing the over-heating strategy; that is, the higher the over-heated bath temperature $T_H$, the shorter the time $\Delta t$ of heating the cylinder from $T_{LT}$ to $T_{HT}$. Experimentally, Fig 5a shows that when the over-heated bath temperature $T_{HIGH}$ is raised to 115°C from a temperature of 95°C, the time t1 for the reactor 15 to reach the 95°C ($T_{HT}$) from the 50°C ($T_{LT}$) is much shorter than the time t2 for the reactor 15 to reach 95°C ($T_{HT}$) when the bath medium 75 is set at 95°C.

Similarly, the time of cooling the cylinder from $T_{HT}$ to $T_{LT}$, $\Delta t$, is determined to be

$$\Delta t = \frac{Q_{total}}{\dot{Q}_{avg}} = \frac{Vc_p\,(T_{HT} - T_{LT})}{A_s[0.5(T_{HT} + T_{LT}) - T_{LOW}]0.683\frac{k}{D}\left(\frac{\rho D}{\mu}\right)^{0.466}P_r^{1/3}v^{0.466}} \quad \dots\dots\dots\dots\dots\dots\dots Eq\ (7)$$

Eq (7) shows that advantage of implementing the under-heating strategy; that is, the lower the under-heated bath temperature $T_{LOW}$, the shorter the time $\Delta t$ of cooling the cylinder from $T_{HT}$ to $T_{LT}$. Experimentally, Fig 5b shows that when the under-heated bath temperature $T_{LOW}$ is reduced to 20°C from a temperature of 50°C, the time t3 for the reactor 15 to reach 50°C ($T_{LT}$) from 95°C ($T_{HT}$) is much shorter than the time t4 for the reactor 15 to reach the 50°C ($T_{LT}$) when the bath is set at the 50°C.

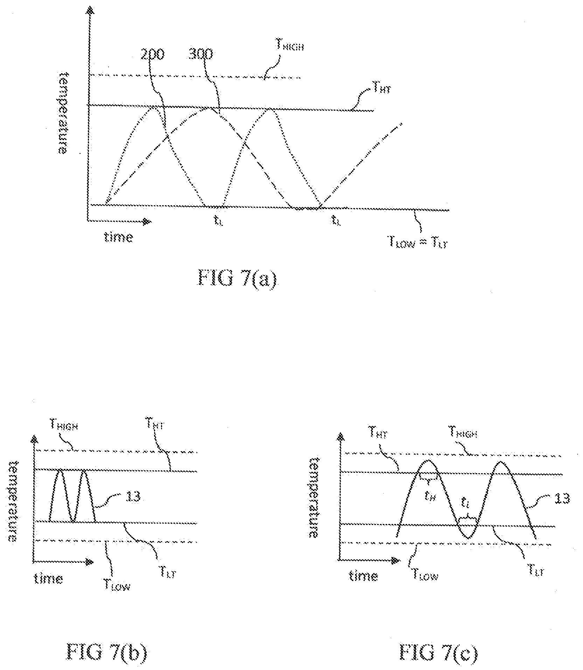

Various exemplary methods for conducting rapid thermal cycling and nucleic acid processing using the apparatus described above are described as follows. When performing thermal cycling by transferring the reactors 15 alternately between a high temperature bath 50 and a low temperature bath 51, **Fig 7(a)** shows the reactor temperature variation curve 200 (dotted curve) in a preferred embodiment of a method in this invention using the over-heating feature in the high temperature bath 50 and the temperature variation curve 300 (dashed curve) in another embodiment without using the overheating method in the high temperature bath 50. Thus, during thermal cycling the temperature ramping up rate of the reactor 15 is faster for the curve 200 than for the curve 300. Herein, the high temperature bath 50 is set at a temperature $T_{HIGH}$ that is higher than the $T_{HT}$ as required for the denaturation temperature and the low temperature bath 51 is set at the $T_{LT}$ that is suitable for annealing and/or extension. After the baths are heated to the respective bath temperatures $T_{HIGH}$ and $T_{LT}$, the reactor transfer mechanism 85 transfers the reactors 15 to the high temperature bath 50 to initiate the thermal cycling for the PCR or other amplification reaction with or without a hot start step which may or may not require a separate bath of a suitable temperature for the hot start step. Once the reactor temperature corresponding to the curves 200 or 300 reaches the $T_{HT}$ in the high temperature bath 50, the reactor transfer mechanism 85 pulls out the reactors 15 and transfers them to the low temperature bath 51 for the reactors 15 to reach $T_{LT}$, and subsequently transfers the reactors 15 back to the high temperature bath 50 for thermal cycling. In this embodiment, the reactor 15 is allowed to stay in the bath 51 for a period of time, called residence time $t_L$ as required for nucleic acid amplification. Once the residence time $t_L$ is reached in the bath 51, the reactor transfer mechanism 85 lifts up the reactors 15 out of the bath 51 to transfer to the bath 50 to continue the thermal cycling. Similar residence time may also be applied in the high temperature bath 50 (not shown). In one embodiment, the residence time $t_L$ can be measured by the reactor temperature monitoring unit 34 in the apparatus that moves together with the reactor holder 33 as shown in Fig. 1a. However, this method may be costly to implement. When the reactors 15 plunge into a bath, the reactor temperature curves 200 or 300 can change at an extremely high rate, such as 40 degree Celsius to 90 degree Celsius per second. To control precisely the timing of lifting the reactor 15 by the reactor transfer mechanism 85, it requires a very fast temperature sampling and signal processing electronics, fast data communication with the reactor transfer mechanism 85, and very responsive mechanical motion components such as motors and actuators in the reactor transfer mechanism 85. But practically, such a fast electronic device may not be available or is too costly to a particular entity making the present apparatus. In another embodiment, the residence time $t_L$ is counted from the moment the reactors 15 are immersed into a bath. For example, the reactor 15 is immersed in a bath for 4 seconds before being moved out of the bath, given an assumption that the reactors 15 have reached the target temperature $T_{LT}$ or its vicinity within 1 second after immersion and stayed at the target temperature $T_{LT}$ or its vicinity for 3 seconds. This method is important since it can be implemented at low cost, without a need for any device capable of high speed temperature sampling, processing and data communication. Similar ultra-rapid heating and cooling is observed also for plastic and metal reactors 15 of various shapes. For plastic reactors 15, we particularly use a $T_{LT}$ substantially below the room temperature using active cooling devices such as thermoelectrical coolers. The apparatus may allow user selection for the conditions $T_{HIGH} = T_{HT}$ or $T_{LOW} = T_{LT}$ if desired.

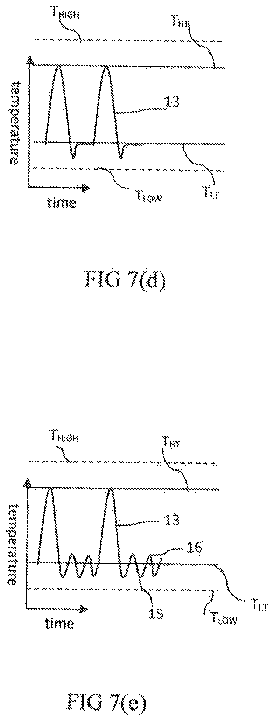

**Fig 7(b)** describes a method of conducting thermal cycling using over-heating in the high temperature bath 50 and under-heating in the low temperature bath 51. The temperature $T_{LOW}$ in the low temperature bath 51 is set below the $T_{LT}$ so that the heat transfer between the reactor 15 and the bath medium 75 in the low temperature bath 51 is much stronger, leading to a much more rapid cooling of the reactor 15. When the reactor temperature reaches the $T_{LT}$, the reactor transfer mechanism 85 lifts up the reactors 15 immediately and transfers them to the high temperature bath 50 to continue thermal cycling. **Fig 7(c)** further describes a thermal cycling method with the reactor temperature overshooting above the $T_{HT}$ and below the $T_{LT}$. This method enables the reactors 15 to stay in the vicinity of the $T_{HT}$ for a longer period of time $t_H$ for denaturation and/or to stay in the vicinity of the $T_{LT}$ for a longer period of time $t_L$ for annealing and extension. In another embodiment shown in **Fig 7(d),** after the reactors 15 reach a temperature in a vicinity of the $T_{LT}$, the reactors 15 are moved out of the low temperature bath 51 into a zone of air to maintain the reactor temperature 13 in the vicinity or at $T_{LT}$ for a time period required for nucleic acid analysis such as annealing and/or extension in PCR, and then the reactors 15 are moved to the high temperature bath 50 to continue thermal cycling. Air in the air zone can be heated and the heated air zone can be established near the low temperature bath 51 using an infrared heater, a fan blowing hot air, an electrical resistance wire heater, or be a simple air zone at a room temperature. The heated air zone can also be created above the bath medium 75 within the either high temperature bath 50 or low temperature bath 51 to utilize the confined space in the baths 50, 51 for better maintenance of reactor temperature 13. The heated air zone can also be created above the bath medium 75 at the opening of the either high temperature bath 50 or low temperature bath 51. The same strategy can also be applied to the high temperature bath 50 for maintaining the $T_{HT}$ for a prescribed period of time.

In another embodiment shown in **Fig 7(e),** after the reactors 15 reach below the $T_{LT}$ in the low temperature bath 51, the reactor transfer mechanism 85 moves the reactors 15 out of the bath 51 into a heated air zone to heat the reactors 15 above the $T_{LT}$, and subsequently moves the reactors 15 into the low temperature bath 51 to cool the reactors 15 below the $T_{LT}$, and repeats this operation as many times as required to generate an oscillatory temperature variation around the $T_{LT}$ for annealing and primer extension. The same strategy can also be applied to the high temperature bath 50 by using a cooled air zone for maintaining the $T_{HT}$ for a prescribed period of time.

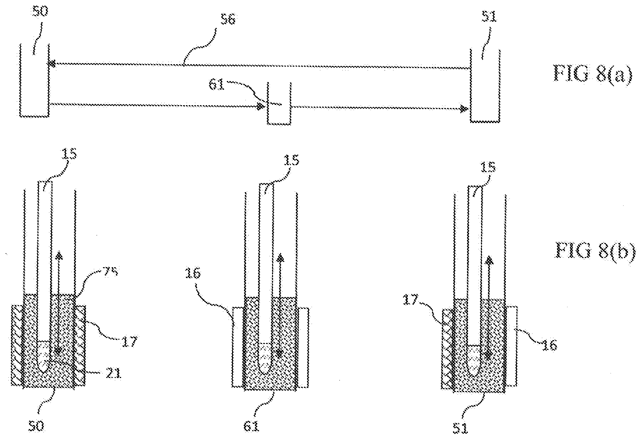

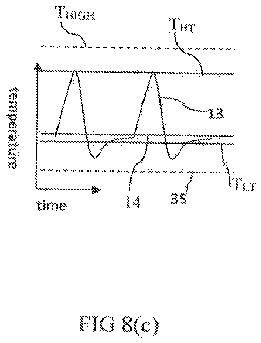

**Fig 8(a)** shows another embodiment of thermal cycling by rapidly cooling the reactors 15 using one under-heating bath 61 which is maintained at a temperature 35 below the $T_{LT}$. The reactor 15 is then moved to the low temperature bath 51 to maintain the reactor temperature 13 in the vicinity of the $T_{LT}$. During thermal cycling along reactor transfer path 56, the reactors 15 leave the high temperature bath 50 which could be under an over-heating condition and move into the under-heating bath 61 to rapidly reach to the vicinity of the $T_{LT}$, and then move into the low temperature bath 51 to sustain at the $T_{LT}$ for a required period of time and carry out fluorescent imaging of the reactors 15 for conducting real-time quantitative PCR, before moving the reactors 15 back to the high temperature bath 50 for thermal cycling. The above described fluorescent imaging of the reactors 15 at every cycle of the thermal cycling may not be necessary for every application. For some applications, the fluorescent imaging of the reactors 15 can be carried out at every few cycles in order to reduce the total time of nucleic acid analysis. The fluorescent imaging of the reactors 15 can be carried out at the end of the thermal cycling for conducting end-point PCR detection. **Fig 8 (b)** shows the schematics of the high temperature bath 50, under-heating bath 61, and the low temperature bath 51 in this embodiment. The under-heating bath 61 can also be cooled to below a room temperature. The lower the temperature of the under-heating bath 61, the faster the cooling of the reactors 15 to the vicinity of the $T_{LT}$. Before the reactors 15 are moved out of the under-heating bath 61, the reactor temperature 13 can be same or lower or higher than the $T_{LT}$. It may be difficult to cool the reactors 15 exactly to the $T_{LT}$ in the under-heating bath 61 before transferring to the low temperature bath 51. Such difficulties include availability of a temperature controller capable of high sampling rate from temperature sensor, fast temperature signal processing, fast temperature data transfer to temperature controller or a signal processing controller through a communication port, a time lag for the motion mechanism to move the reactors 15 out of the low temperature bath 51, and the like. Therefore, it is more realistic to have the reactors 15 to be cooled to the vicinity of the $T_{LT}$ with a temperature range of such vicinity to be 0.5-5°C below or above the $T_{LT}$ in the under-heating bath 61 before transferring to the low temperature bath 51. To speed up the temperature change to reach the $T_{LT}$ after moving the reactors 15 from the under-heating bath 61 to the low temperature bath 51, the low temperature bath 51 can be set at a temperature different from the $T_{LT}$. For example, if the reactors 15 in the under-heating bath 61 of temperature 35 is cooled to below the $T_{LT}$, the temperature 14 in the low temperature bath 51 can be set higher than the $T_{LT}$, as shown in **Fig 8(c).** Similarly, if the reactors 15 in the under-heating bath 61 is cooled to above the $T_{LT}$, the temperature 14 in the low temperature bath 51 can be set lower than the $T_{LT}$. Specifically, the temperature in the low temperature bath 51 can be in the vicinity of the $T_{LT}$. Choice of bath medium temperature in the under-heating bath 61 can vary according the heat transfer characteristics of the reactors 15. For example, for the reactors 15 made of glass or metal materials, the temperature 35 of the under-heating bath 61 can be set at room temperature or in its vicinity, whereas for the reactors 15 made of plastics, the temperature 35 of the under-heating bath 61 can be set substantially lower than a room temperature, or at a room temperature. The above temperature setting is also dependent on the volume of the reactors 15 and wall thickness of the reactors 15. For large reactors 15 and the reactors 15 made of plastics, the under-heating bath 61 can be actively cooled by thermoelectric coolers or a recirculation liquid cooling system to reach a very low bath temperature such as slightly above 0°C to 20°C. With the use of anti-freezing agent or use of salt, sub-zero liquid can be used in the under-heating bath 61 to further enhance the reactor cooling rate. The above intense under-heating or cooling means not only cool the plastic reactors 15 rapidly, but also cool the glass or metal or plastic-metal reactors 15 rapidly. Moreover, when the thermal cycling scheme shown in Fig 7 (c) incorporates an over-heating bath for rapid heating to the $T_{HT}$, the entire thermal cycling process can be further shortened. Furthermore, to shorten the time after transferring the reactors 15 from the under-heating bath 61 to the low temperature bath 51, the low temperature bath 51 can be set at a temperature higher than the $T_{LT}$, which allows the reactor temperature rise above the $T_{LT}$ to achieve a required period of time in the vicinity of the $T_{LT}$, or carry out the cyclic transfer of the reactors 15 between the under-heating bath 61 and the low temperature bath 51, similar to the process shown in Fig 6(e), to achieve a required period of time in the vicinity of the $T_{LT}$. In the above scheme, the under-heating bath 61 can be maintained at a room temperature or below a room temperature or above a room temperature. The same strategy of maintaining the reactor temperature 13 at a target temperature for a period of time shown in Figs 7(a, b, c) can be applied to the high temperature bath 50 for maintaining reactor temperature 13 at the $T_{HT}$ for a period of time.

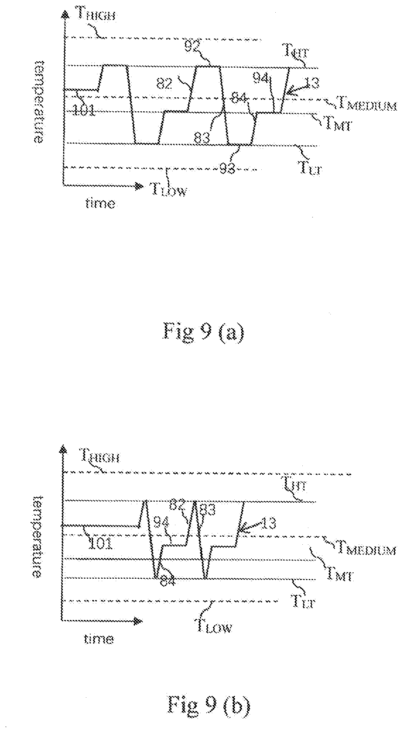

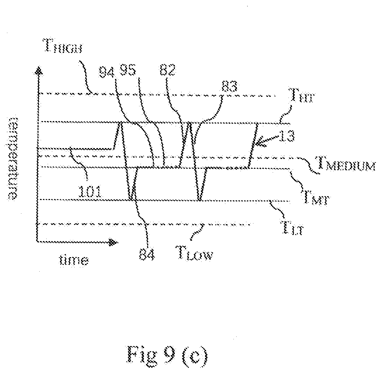

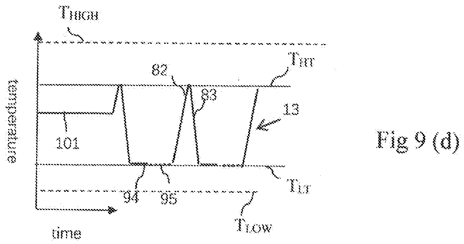

**Fig 9(a)** describes an embodiment with deployment of stabilization baths (a 3-step PCR). The reactor temperature 13 is ramping up 82 in the high temperature bath 50 when set at temperature $T_{HIGH}$. The reactor temperature 13 is stabilizing 92 is when the reactor 15 is in the stabilization bath at $T_{HT}$. The reactor temperature 83 is ramping down in the low temperature bath 51 set at temperature $T_L$. The reactor temperature 13 is stabilizing 93 when the reactor 15 is in the stabilization bath at $T_{LT}$. The reactor temperature 13 is ramping up 84 to the medium temperature at $T_{MEDIUM}$. The reactor temperature 94 is when the reactor 15 is in the stabilization bath for the reactor 15 to maintain at the $T_{MT}$. Fig 9(b) describes another embodiment of a 3-step PCR as in Fig 8(a) without deployment of stabilization baths at $T_{HT}$ and $T_{LT}$. Fig 9(c) describes another embodiment of a 3-step PCR with deployment of stabilization baths where the bath medium 75 in different baths are different. For example, the bath medium 75 in the high temperature bath 50 can be thermally conductive particles or a liquid having high evaporative temperature such as over 100 degree Celsius and the bath medium 75 in the low temperature bath 51 is pure water. The reactor temperature 13 is ramping up 82 in the high temperature bath 50 when set at temperature $T_{HIGH}$ and containing metal powder or liquid. The reactor temperature 13 is ramping down 83 in the low temperature bath 51 set at temperature $T_{LOW}$ and containing metal powder or liquid. The reactor temperature 13 is ramping up 84 in the medium temperature or high temperature bath set at temperature $T_{MEDIUM}$, containing metal powder or liquid. The reactor temperature 13 is when the reactor 15 is stabilizing 94 in the stabilization bath for the reactor 15 to maintain at the medium target temperature $T_{MT}$, with the stabilization bath containing metal powder or liquid or heated air. The reactor temperature 13 is achieved when the reactor 15 is stabilizing 95 in another stabilization bath containing heated air or an air zone outside the stabilization bath for taking at least one fluorescent image of the reactor 15. **Fig 9(d)** illustrates the method of Fig. 8(c) but without the bath set at temperature $T_{MEDIUM}$ and the stabilizings 94, 95 being conducted at $T_{LT}$

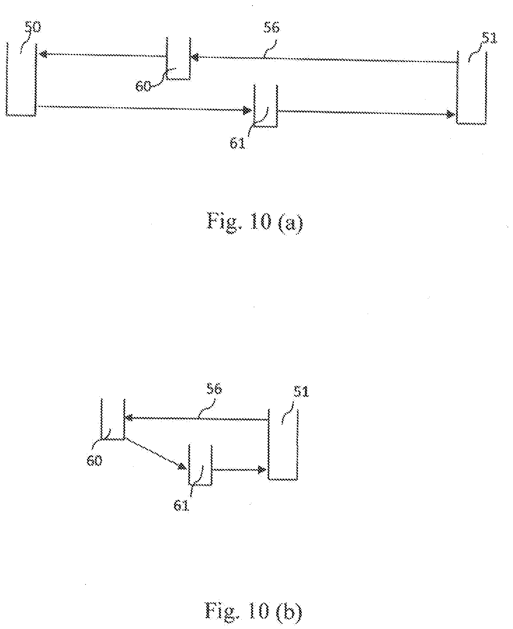

**Fig 10(a)** shows another embodiment in which an over-heating bath 60 is added to the path 56 for transferring the reactors 15 during thermal cycling to speed up the temperature ramping to $T_{HT}$ in the over-heating bath 60. During thermal cycling, the reactors 15 leave the low temperature bath 51 and move into the over-heating bath 60 to rapidly reach to the vicinity of $T_{HT}$, and then move into the high temperature bath 50 to sustain in the vicinity of the $T_{HT}$ for a required period of time before moving back to the low temperature bath 51 and optionally via the under-heating bath 61 before moving to the low temperature bath 51. The higher the temperature of the over-heating bath 60, the faster is the heating of the reactors 15 to the vicinity of $T_{HT}$. Preferably, the temperature in the over-heating bath 60 is between 100degree Celsius to 135degree Celsius. Similar to the bath setup and temperature control described in Figs 7a-c, the high temperature bath 50 can be set at a temperature different from the $T_{HT}$. Specifically, the temperature in the high temperature bath 50 can be in the vicinity or outside of $T_{HT}$. **Fig 10(b)** shows another embodiment in which the high temperature bath 50 is removed from embodiment in Fig 9(a). During thermal cycling in this embodiment, the reactors 15 leave the low temperature bath 51 and move into the over-heating bath 60 to rapidly reach to the vicinity of the $T_{HT}$, and then move back to the low temperature bath 51 and optionally via the under-heating bath 61 before moving to the low temperature bath 51.

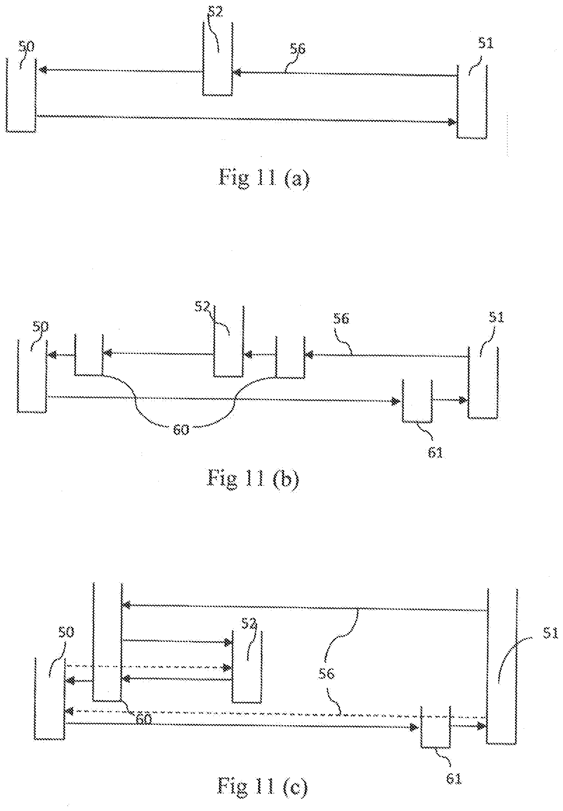

**Fig 11(a)** shows a method to perform biological or biochemical reactions involving 3 temperatures, including the 3-step PCR, in which three heating baths are assembled in a path 56 for transferring the reactors 15 for thermal cycling. The three baths include a high temperature bath 50 for denaturation, a mid temperature bath 52 for primer extension, and a low temperature bath 51 for annealing. **Fig 11(b)** shows that additional under-heating bath 61 is optionally added into the thermal cycling path 56 before the low temperature bath 51, and an over-heating bath 60 is optionally added into the thermal cycling path 56 each before the mid temperature bath 52 and the high temperature bath 50, respectively. **Fig 11(c)** shows an embodiment modified from embodiment shown in Fig 10(b), in which the over-heating bath 60 added before the mid temperature bath 52 is removed from the thermal cycling path 56. During thermal cycling in this embodiment, the reactors 15 leave the low temperature bath 51 and enter the over-heating bath 60 set for high temperature bath 50 or enter the high temperature bath 50, in dashed line to rapidly reach to the vicinity of the mid target temperature 5 as shown in Fig 8(a), and then move to the mid temperature bath 52 to stabilize at the mid target temperature 5 for a required period of time for extension. Once the extension is completed, the reactors 15 move to the over-heating bath 60 to rapidly reach to the vicinity of the $T_{HT}$, and then move to high temperature bath 50 for a required period of time for denaturation, before moving back to the low temperature bath 51 or optionally via the under-heating bath 61 before moving to the low temperature bath 51. **Fig 11(d)** shows an embodiment modified from embodiment shown in Fig 10(c), in which the low temperature bath 51 and the high temperature bath 50 are moved. During thermal cycling in this embodiment, the reactors 15 enter the under-heating bath 61, and once the reactors 15 reach the $T_{LT}$ as shown in Fig 8(b), the reactors leave the under-heating bath 61 and enter the over-heating bath 60 set for high temperature bath 50 to rapidly reach to the vicinity of the mid target temperature 5 as shown in Fig 8(b), and then move to the mid temperature bath 52 to stabilize at the mid target temperature 5 for a required period of time for extension. Once the extension is completed, the reactors 15 move to the over-heating bath 60 to rapidly reach to the vicinity of the $T_{HT}$, before moving back to the under-heating bath 61. In the embodiment described in Figures 10 to 11, the reactors 15 can be imaged for fluorescent detection inside one of the bath 51, 52, 60, and 61, if the bath medium 75 inside is transparent to light transmission and has low auto-fluorescence, and such bath medium 75 can be water, water-glycerol mixture, oil, and heated or un-heated air. If the bath medium 75 inside bath 51, 52, 60, and 61 are opaque to light transmission or has high auto-fluorescence, the reactors 15 can be moved to a zone of heated or un-heated air outside the baths or above the bath medium inside the bath for fluorescent imaging. The methods of rapidly transferring the reactors 15 among the heat baths set at different temperatures for nucleic acid samples can be incorporated with additional at least one pre-process like reverse transcription and/or isothermal amplification and/or primer extension. **Fig 12** shows a method to perform biological or biochemical reactions involving at least one pre-heating bath 53 before starting PCR. The at least one pre-heating bath 53 can be set at a temperature suitable for reverse transcription and/or isothermal amplification and/or primer extension. After exposing the reactors 15 to the pre-heating bath 53, the reactors are transferred to other bath for isothermal DNA process step and/or PCR thermal cycling. In the embodiment shown, many baths are optional depending on nucleic acid processing requirement, and the optional baths including the over-heating baths 60, the under-heating bath 61, and the mid temperature bath 52. In another embodiment, no over-target heating method, i.e., neither over-heating nor under-heating, is used. In this embodiment, the baths are set at the target temperature required for nucleic acid analysis or processing. In this embodiment, one or more than one of the following means are used:

1) the reactor moves at high speed in a reciprocating manner inside at least one bath,
2) reactor transfer mechanism 85 transfers the reactor from one bath to another in less than 4 seconds, and preferably less than 1 second,
3) the baths have a high-aspect-ratio geometry with bath heaters 17 being deployed on the bath surfaces forming a shorter bath dimensions, and
4) the bath 51 having a lower temperature has an optically transparent window 27 for fluorescent imaging of liquid sample inside the reactor 15 situated inside the bath 51.

Small reactors 15 with narrow internal cavities such as a small bore glass capillary are difficult for loading sample and reagent liquid by a normal pipette since air can be trapped underneath the liquid inside the reactor cavity. The following means can be used to load the liquid into such narrow cavities: 1) centrifuging the liquid dispensed to the entrance of the reactor cavity, 2) inserting a tube thinner than the internal passage of the cavity down to the bottom of the cavity, 3) using a vacuum to remove air inside the cavity before loading the liquid, 4) using a pre-vacuumed cavity to load the liquid, and 5) using a cavity with at least one vent when loading the liquid, and sealing the vent. After loading the liquid, the loading ports are to be sealed before thermal cycling starts.

Different bath may contain different bath medium 75 for specific advantages as desired. The reactors 15 may be made up of plastics, elastomer, glass, metal, ceramic and their combinations, in which the plastics include polypropylene and polycarbonate. The glass reactor 15 can be made in a form of a glass capillary of small diameters such as 0.1mm-3mm OD and 0.02mm-2mm ID, and the metal can be aluminum in form of thin film, thin cavity, and capillary. Reactor materials can be made from non-biological active substances with chemical or biological stability. At least a portion of the reactor 15 is preferred to be transparent. In another embodiment, the reactors 15 can be in a form of a reactor array chip or a microfluidic reactor chip or arrayed chip. For example, the reactors 15 can be in a form of wells or channels of a substrate plate and optionally covered with a solid layer of material to form closed reaction chambers, in which the reaction material 21 is situated.

The reaction material 21 in all the reactors 15 in the reactor holder 33 may not be identical. Simultaneous PCR can be advantageously conducted for different materials if the bath temperatures are suitable. At least part of the reactor wall may be made of metal sheet of thickness 1 $\mu$m - 2 mm. This feature enhances the rate of heat transfer between the bath and the reaction material 21. At least part of the reactor wall may be made of plastic or glass sheet of thickness 0.5 $\mu$m - 500 $\mu$m. At least a part of the reactor wall is made of transparent material so as to enable the imaging and detection process.

The invention is equally applicable for a single reactor 15 or multiple reactors 15 in the reactor holder 33. The term 'liquid' used in the above description is a general term for the heating medium. For this invention, the heating medium may be in different forms, such as liquid, water, water mixed with solvent or other chemical fluid or other solid particles, solid particles, metal particles, copper particles and powders.

In order to further explain and clearly understand the invention, some examples and illustration below are combined to further instruct the invention, especially of the methods to use the apparatus to carry out the sample testing.

Example 1.1: A method for nucleic acid analysis, comprising the following steps: adding at least one kind of reaction material 21 into at least one reactor 15, sealing the reactor 15, amplifying nucleic acid, and during the nucleic acid amplification, employing over-heating and/or under-heating method, which describes that the temperature of at least one heating bath is higher or lower than the target temperature required for nucleic acid amplification. The reactor 15 is alternately arranged in at least two heating baths with different temperatures for thermal cycle when the nucleic acid is amplified. When the temperature of the reactor 15 comes close or equal to the target temperature required for the nucleic acid amplification, the reactor 15 is moved from the heating bath where the reactor 15 is in to another heating bath quickly. When the over-heating and/or under-heating method adopts the three-step method for nucleic acid amplification, the temperature of the heating bath I is set to higher than the target temperature required for pre-denaturation and denaturation, and the temperature of the heating bath II is set to lower than the target temperature required for denaturation, and the temperature of the heating bath III is set to higher than the target temperature required for extension, and the thermal cycle of the reactor is alternately carried out according to an order of the heating bath I, the heating bath II, the heating bath III, the heating bath I.

Example 1.2: The difference between the present example and example 1.1 is that when the temperature of the reactor 15 achieves the target temperature required for the nucleic acid amplification, the reactor 15 is moved from the heating bath I and transferred to the heating bath II quickly.

Example 1.3: The difference between the present example and example 1.1 is that when the temperature of the reactor 15 exceeds the target temperature required for the nucleic acid amplification, the reactor 15 is quickly moved from the heating bath III in to the heating bath IV, and the temperature of the heating bath IV is set to the target temperature required for extension.

Example 2.1: The difference between the present example and example 1.1 is that it also comprises reverse transcription (Reverse Transcription-Polymerase Chain Reaction) before the nucleic acid amplification.

Example 2.2: The difference between the present example and example 2.1 is that the reverse transcription is carried out prior to the thermal cycle of the nucleic acid amplification or simultaneously.

Example 2.3: The difference between the present example and example 2.2 is that the reverse transcription is carried out prior to the nucleic acid amplification, which needs to add a heating bath or to set the temperature of the heating bath to the target temperature required for the reverse transcription and then to set the temperature of heating bath to the target temperature required for nucleic acid amplification.

Example 3.1: The difference between the present example and example 1.1 is that when the overtemperature method adopts the three-step method for nucleic acid amplification, the temperature of the 1st heating bath is set to higher than the target temperature required for pre-denaturation and denaturation, the temperature of the heating bath II is set to the target temperature required for pre-denaturation and denaturation, and the temperature of the heating bath III is set to lower than the target temperature required for annealing, and the temperature of the heating bath IV is set to the target temperature required for annealing, and the temperature of the heating bath V is set to the target temperature required for extension, and the thermal cycle of the reactor 15 is alternately carried out among the heating baths with different temperatures according to an order of the heating bath I, the heating bath II, the heating bath III, the heating bath IV, the heating bath V, the heating bath I, and so on.

Example 3.2: The difference between the present example and example 1.1 is that the over-temperature method is used to carry out the polymerase chain reaction, and the reactor 15 is alternately placed in the heating baths with different temperatures. The heating baths is respectively heating bath I, heating bath II, heating bath III, heating bath IV, heating bath V, heating bath VI. A selected temperature in the heating bath I is 105-135 °C. A selected temperature of the heating bath II is 95 °C. A selected temperature of the heating bath III is 10-40 °C. A selected temperature of the heating bath IV is 50 °C. A selected temperature of the heating bath V is 82-112 °C. A selected temperature of the heating bath VI is 72 °C. The selected temperature of the heating bath I is higher than the target temperature required for pre-denaturation and denaturation, the selected temperature of the heating bath III is lower than the target temperature required for annealing, and the selected temperature of the heating bath V is higher than the target temperature required for extension. The temperature of the reactor in the heating bath I will achieve, come close to or exceed the target temperature 95 °C for a few seconds, and the reactor is moved from the heating bath I to the heating bath II quickly. The temperature of the reaction system in the heating bath III will achieve, come close to or exceed the target temperature 50 °C for a few seconds, and then the reactor is moved to the heating bath IV quickly. The temperature of the reaction system in the heating bath V will achieve, come close to or exceed the target temperature 72 °C for a few seconds, and then the reactor 15 is moved to the heating bath VI quickly. Then the reactor 15 is moved in turn to the heating bath I, the heating bath II, the heating bath III, the heating bath IV, the heating bath V, heating bath VI, the heating bath I according to motion track A , and with such 35 cycles, the whole process needs just a few minutes. Of course, the transfer here can be artificial one, it also can be mechanized done using the reactor transfer mechanism 85 shown in Fig 1. No matter which kind, compared with the ordinary PCR, this kind of method is time-saving, high-speed, high-efficiency and is suitable for the detection in emergence. If using PCR instrument, the process is divided into 95 °C pre-denaturation 4 min, 95 °C denaturation 1 min, 50 °C annealing 1 min, 72 °C extension 1.5 min, 72 °C extension 5 min, 95 °C denaturation 1 min, 50 °C annealing 1 min, 72 °C extension 1.5 min for 35 cycles, and the last is kept in 4 °C. The whole process takes nearly two hours and is slow-speed and low-efficiency.

Example 3.3: The difference between the present example and example 1.1 is that, in any of the above schemes, preferably, when the overtemperature method adopts the two-step method for nucleic acid amplification, the temperature of the heating bath I is set to higher than the target temperature required for pre-denaturation and denaturation and the temperature of the heating bath II is set to lower than the target temperature required for annealing or extension.

Example 3.4: The difference between the present example and example 3.3 is that when the over-temperature method adopts the two-step method for nucleic acid amplification, the temperature of the heating bath I is set to higher than the target temperature required for pre-denaturation and denaturation, the temperature of the heating bath II is set to lower than the target temperature required for annealing, the temperature of the heating bath III is set to the target temperature required for annealing, and the reactor 15 transfers from the heating bath I to the heating bath II and transfers quickly to the heating bath III as the temperature lower than the target temperature required for annealing and transfers to the heating bath I after a certain time for alternate circulation, and the thermal cycle step of nucleic acid amplification is the heating bath I, the heating bath II, the heating bath III, then the heating bath I1.

From the foregoing description it will be understood by those skilled in the art that many variations or modifications in details of design, construction and operation may be made without departing from the present invention as defined in the claims.

**Claims**

1.  An apparatus for thermal processing of nucleic acid in a thermal profile, the apparatus employing bath media (75) and a reactor holder (33) for holding reactor(s) (15) each accommodating reaction material (21) containing the nucleic acid and the reactor(s) (15) being in any form such as tube(s) or wellplate(s) or chip(s) or cartridge(s), the apparatus comprising:

    a first bath (50);
    a second bath (51),
    the bath mediums (75) are provided in the first bath (50) and the second bath (51) respectively, wherein the bath media (75) in the first bath (50) and the second bath (51) respectively are maintainable at two different temperatures $T_{HIGH}$ and $T_{LOW}$; and a transfer means (85) configured to make the reactor(s) (15) to be in the two baths (50, 51) in a plurality of thermal cycles to alternately attain:

      - a predetermined high target temperature $T_{HT}$, and
      - a predetermined low target temperature $T_{LT}$,

    while the apparatus is configured to adapt to a temperature-offset feature defined by at least one condition from the group consisting:

      a) the $T_{HT}$ is lower than the $T_{HIGH}$, and
      b) the $T_{LT}$ is higher than the $T_{LOW}$,

the transfer means (85) being operable by at least one mode from the group consisting:

a temperature guided motion controlling means (TeGMCM) that is operable based on the real-time temperature as sensed by a reactor temperature sensor (38) during thermal cycling,
and
a time guided motion controlling means (TiGMCM) that is operable based on the time-periods for which the reactor(s) (15) are allowed to be in the baths (50,51),
the bath medium (75) in any of the baths (50, 51)being in any phase including air, liquid, solid, powder and a mixture of any of these;
**characterized in that** the transfer means (85) is configured to initiate lift-off of the reactor(s) (15) from the bath(s) (50, 51) when the reactor(s) (15) reach a first lift-off temperature that is lower than the $T_{HT}$ and a second lift-off temperature that is higher than the $T_{LT}$ to compensate for operational electro-mechanical delays that unwantedly cause over heating or over cooling of the reactor(s) (15).

2. The apparatus according to claim 1 wherein the TiGMCM can be user calibrated for the time-periods.

3. The apparatus according to claim 1 wherein the bath medium (75) in the first bath (50) is maintained above 100 degrees Celsius.

4. The apparatus according to claim 1 wherein the bath medium (75) in the second bath (51) is maintained below room temperature.

5. The apparatus according to claim 1, wherein a first offset between the $T_{HIGH}$ and the $T_{HT}$ is within a range 1- 400 degree Celsius and a second offset between the $T_{LOW}$ and the $T_{LT}$ is within a range 1- 100 degree Celsius.

6. The apparatus according to claim 1 further comprising bath cover(s) on the bath(s) (50, 51), the cover(s) opening to allow the reactor(s) (15) to be in the bath(s) (50, 51) and closing after the reactor(s) (15) is/are removed from the bath(s) (50, 51).

7. A method for thermal processing nucleic acid in a thermal profile, the method comprising:

employing a reactor holder (33) for holding reactor(s) (15) each accommodating reaction material (21) containing the nucleic acid and the reactor(s) (15) being in any form such as tube(s) or wellplate(s) or chip(s) or cartridge(s);
employing an apparatus comprising a first bath (50) and a second bath (51);
maintaining bath media (75) in the first bath (50) at temperature $T_{HIGH}$ and in the second bath (51) at temperature $T_{LOW}$; and
employing a transfer means (85) in the apparatus to allow the reactor(s) (15) to be in the two baths (50, 51) in a plurality of thermal cycles to alternately attain:

- a predetermined high target temperature $T_{HT}$, and
- a predetermined low target temperature $T_{LT}$; and

adapting to a temperature-offset feature defined by at least one condition from the group consisting:

a. the $T_{HT}$ is lower than the $T_{HIGH}$, and
b. the $T_{LT}$ is higher than the $T_{LOW}$,

the transfer means (85) being operable by at least one mode from the group consisting:

a temperature guided motion controlling means (TeGMCM) that is operable based on the real-time temperature as sensed by a reactor temperature sensor (38) during thermal cycling, and
a time guided motion controlling means (TiGMCM) that is operable based on the times for which the reactor(s) (15) are allowed to be in the baths (50, 51),
the bath medium (75) in any of the baths (50, 51) being in any phase including air, liquid, solid, powder and a mixture of any of these;
**characterized in that** the method further comprises calibrating the transfer means (85) to initiate lift-off of the reactor(s) (15) from the bath(s) (50, 51) when the reactor(s) (15) reach a first lift-off temperature that is lower than the $T_{HT}$ and a second lift-off temperature that is higher than the $T_{LT}$ to compensate for operational electro-mechanical delays that unwantedly cause over heating or over cooling of the reactor(s) (15).

8. The method according to claim 7 further comprising:
maintaining the bath medium (75) in the first bath (50) above 100 degrees Celsius.

9. The method according to claim 7 further comprising:
maintaining the bath medium (75) in the second bath (51) below room temperature.

10. The method according to claim 7 further comprising:

setting a first offset between the $T_{HIGH}$ and the $T_{HT}$ within the range 1- 400 degree Celsius; and
setting a second offset between the $T_{LOW}$ and the $T_{LT}$ within the range 1- 100 degree Celsius.

11. The method according to claim 7 further comprising:
adding a second liquid to a first liquid in the bath medium (75) in the first bath (50), the boiling point of the second liquid being higher than the boiling point of the first liquid.

12. The method according to claim 11, wherein, the boiling point of a mixture of the first liquid and the second liquid is higher than 100 °C.

13. The apparatus according to claim 1, wherein a first offset between the $T_{HIGH}$ and the $T_{HT}$ is within a range 10- 400 degree Celsius and a second offset between the $T_{LOW}$ and the $T_{LT}$ is within a range 10- 100 degree Celsius.

14. The apparatus according to claim 1, further comprising: a further bath wherein the bath medium (75) is liquid or hot air that is maintainable at 40-80 degree Celsius , wherein the bath medium (75) and at least a portion of the sixth bath is transparent to allow transmission of illumination light from a light source and transmission of emitted light from the reactor(s) (15).

15. The method according to claim 8, further comprising:

employing a further bath for annealing and extension, wherein the bath medium (75) is liquid or hot air;
maintaining the bath medium (75) in the sixth bath at 40-80 degree C; and
conducting fluorescence imaging through the sixth bath at the constant temperature, wherein the bath medium and at least a portion of the sixth bath is transparent to allow transmission of illumination light from a light source and transmission of emitted light from the reactor(s) (15).

**Patentansprüche**

1. Einrichtung zur thermischen Verarbeitung von Nukleinsäure in einem thermischen Profil, die Einrichtung verwendet Badmedien (75) und einen Reaktorhalter (33) zum Halten von Reaktor(en) (15), die jeweils Reaktionsmaterial (21) aufnehmen, das die Nukleinsäure enthält, und der (die) Reaktor(en) (15) ist (sind) in einer beliebigen Form wie Rohr(en) oder Brunnenplatte(n) oder Chip(s) oder Kartusche(n), die Einrichtung umfasst:

ein erstes Bad (50);
ein zweites Bad (51),
das Badmedium (75) in dem ersten Bad (50) und dem zweiten Bad (51) jeweils vorgesehen sind, wobei der Badmedien (75) in dem ersten Bad (50) und dem zweiten Bad (51) jeweils bei zwei unterschiedlichen Temperaturen $T_{HOCH}$ und $T_{NIEDRIG}$ aufrechterhalten werden können; und
ein Übertragungsmittel (85), das so konfiguriert ist, dass der (die) Reaktor(en) (15) in den beiden Bäder (50, 51) in einer Vielzahl von Wärmezyklen abwechselnd um Folgendes zu erreichen:

- eine vorgegebene hohe Zieltemperatur $T_{HT}$, und
- eine vorgegebene niedrige Zieltemperatur $T_{LT}$,

während die Einrichtung so konfiguriert ist, dass sie sich an ein Temperaturverschiebungsmerkmal anpasst, das durch mindestens eine Bedingung aus der folgenden Gruppe definiert ist:

EP 3 463 669 B1

a) die $T_{HT}$ ist niedriger als der $T_{HOCH}$, und
b) die $T_{LT}$ ist höher als der $T_{NIEDRIG}$,

das Übertragungsmittel (85) ist durch mindestens einen Modus aus der Gruppe betreibbar, die besteht aus: einem temperaturgeführten Bewegungssteuerungsmittel (TeGMCM), das auf der Grundlage der Echtzeit-Temperatur betreibbar ist, wie sie von einem Reaktor-Temperatursensor (38) während der thermischen Zyklen erfasst wird,
und
ein zeitgeführtes Bewegungssteuerungsmittel (TiGMCM), das auf der Grundlage der Zeitperioden, für die der (die) Reaktor(en) (15) in den Bäder (50, 51) befindet (befinden), betrieben werden kann, das Badmedium (75) in einem der Bäder (50, 51) sich in einer beliebigen Phase befindet, einschließlich Luft, Flüssigkeit, Feststoff, Pulver und einer Mischung daraus;
**dadurch gekennzeichnet, dass** das Übertragungsmittel (85) so konfiguriert ist, dass es das Abheben der (die) Reaktor(en) (15) von dem (den) Bad (Bäder) (50, 51) initiiert, wenn der (die) Reaktor(en) (15) eine erste Abhebetemperatur erreicht (erreichen), die niedriger als die $T_{HT}$ ist, und eine zweite Abhebetemperatur, die höher als die $T_{LT}$ ist, um elektro-mechanische Verzögerungen im Betrieb auszugleichen, die unerwünschte Überhitzung oder Überkühlung des (der) Reaktors (Reaktoren) (15) bewirken.

2. Einrichtung nach Anspruch 1, wobei das TiGMCM vom Benutzer für die Zeitperioden kalibriert werden kann.

3. Einrichtung nach Anspruch 1, wobei das Badmedium (75) im ersten Bad (50) über 100 Grad Celsius aufrechterhalten.

4. Einrichtung nach Anspruch 1, wobei das Badmedium (75) im zweiten Bad (51) unter Raumtemperatur aufrechterhalten.

5. Einrichtung nach Anspruch 1, wobei eine erste Verschiebung zwischen dem $T_{HOCH}$ und dem $T_{HT}$ innerhalb eines Bereichs von 1-400 Grad Celsius und eine zweite Verschiebung zwischen dem $T_{NIEDRIG}$ und dem $T_{LT}$ innerhalb eines Bereichs von 1-100 Grad Celsius liegt.

6. Einrichtung nach Anspruch 1, weiterhin umfasst der (die) Badabdeckung(en) auf dem (den) Bad (Bäder) (50, 51), der (die) Abdeckung(en) öffnet (öffnen), um den (die) Reaktor(en) (15) in dem (den) Bad (Bäder) (50, 51) zu ermöglichen, und schließt (schließen) sich, nachdem der (die) Reaktor(en) (15) aus dem (den) Bad (Bäder) (50, 51) entfernt worden ist (sind).

7. Verfahren zur thermischen Prozessieren von Nukleinsäure in einem thermischen Profil, das Verfahren umfasst:

Verwendung eines Reaktorhalters (33) zum Halten von Reaktor(en) (15), die jeweils Reaktionsmaterial (21) aufnehmen, das die Nukleinsäure enthält, und der (die) Reaktor(en) (15) ist (sind) in einer beliebigen Form wie Rohr(en) oder Brunnenplatte(n) oder Chip(s) oder Kartusche(n);
Verwendung einer Einrichtung umfassend ein erstes Bad (50) und ein zweites Bad (51);
Aufrechterhaltung der Badmedien (75) im ersten Bad (50) bei der Temperatur $T_{HOCH}$ und im zweiten Bad (51) bei der Temperatur $T_{NIEDRIG}$; und
Verwendung eines Übertragungsmittels (85) in der Einrichtung, um zu ermöglichen, dass sich der (die) Reaktor(en) (15) in den beiden Bädern (50, 51) in einer Vielzahl von Wärmezyklen abwechseln, um Folgendes zu erreichen:

- eine vorgegebene hohe Zieltemperatur $T_{HT}$, und
- eine vorgegebene niedrige Zieltemperatur $T_{LT}$; und

Anpassung an ein Temperaturverschiebungsmerkmal, das durch mindestens eine Bedingung aus der folgenden Gruppe definiert ist:

a. die $T_{HT}$ ist niedriger als der $T_{HOCH}$, und
b. die $T_{LT}$ ist höher als der $T_{NIEDRIG}$,

das Übertragungsmittel (85) ist durch mindestens einen Modus aus der Gruppe betreibbar, die besteht aus::

ein temperaturgeführtes Bewegungssteuerungsmittel (TeGMCM), das auf der Grundlage der Echtzeit-Temperatur betrieben werden kann, wie sie von einem Reaktor-Temperatursensor (38) während der thermischen Zyklen erfasst wird, und

ein zeitgeführtes Bewegungssteuerungsmittel (TiGMCM), das auf der Grundlage der Zeiten, für die sich der (die) Reaktor(en) (15) in den Bäder (50, 51), befinden dürfen, betrieben werden kann.

das Badmedium (75) in einem der Bäder (50, 51) sich in einer beliebigen Phase befindet, einschließlich Luft, Flüssigkeit, Feststoff, Pulver und einer Mischung daraus;

**dadurch gekennzeichnet, dass** das Verfahren weiterhin das Kalibrieren der Übertragungsmittel (85) umfasst, um das Abheben des (der) Reaktors (Reaktoren) (15) von dem Bad (den Bäder) (50, 51) zu initiieren, wenn der (die) Reaktor(en) (15) eine erste Abhebetemperatur erreicht (erreichen), die niedriger als die $T_{HT}$ ist, und eine zweite Abhebetemperatur, die höher als die $T_{LT}$ ist, um elektro-mechanische Verzögerungen im Betrieb auszugleichen, die unerwünschte Überhitzung oder Überkühlung des (der) Reaktors (Reaktoren) (15) bewirken.

8. Verfahren nach Anspruch 7 weiterhin umfassend:
Aufrechterhaltung des Badmediums (75) im ersten Bad (50) über 100 Grad Celsius.

9. Verfahren nach Anspruch 7 weiterhin umfassend:
Aufrechterhaltung des Badmediums (75) im zweiten Bad (51) unter Raumtemperatur.

10. Verfahren nach Anspruch 7 weiterhin umfassend:

Einstellung eines ersten Satzes zwischen dem $T_{HOCH}$ und dem $T_{HT}$ innerhalb des Bereichs von 1- 400 Grad Celsius; und
Einstellung eines zweiten Satzes zwischen dem $T_{NIEDRIG}$ und dem $T_{LT}$ im Bereich von 1 - 100 Grad Celsius.

11. Verfahren nach Anspruch 7 weiterhin umfassend:
Zugabe einer zweiten Flüssigkeit zu einer ersten Flüssigkeit im Badmedium (75) im ersten Bad (50), der Siedepunkt der zweiten Flüssigkeit ist höher als der Siedepunkt der ersten Flüssigkeit.

12. Verfahren nach Anspruch 11, wobei der Siedepunkt einer Mischung aus der ersten und der zweiten Flüssigkeit höher als 100°C ist.

13. Einrichtung nach Anspruch 1, wobei eine erste Verschiebung zwischen dem $T_{HOCH}$ und dem $T_{HT}$ in einem Bereich von 10-400 Grad Celsius und eine zweite Verschiebung zwischen dem $T_{NIEDRIG}$ und dem $T_{LT}$ in einem Bereich von 10-100 Grad Celsius liegt.

14. Einrichtung nach Anspruch 1, weiterhin umfassend: ein weiteres Bad, wobei das Badmedium (75) flüssig oder heiße Luft ist, die bei 40-80 Grad Celsius aufrechterhalten werden kann, wobei das Badmedium (75) und mindestens ein Teil des sechsten Bades transparent ist, um die Übertragung von Beleuchtungslicht von einer Lichtquelle und die Übertragung von emittiertem Licht von dem (den) Reaktor(en) (15) zu ermöglichen.

15. Verfahren nach Anspruch 8, weiterhin umfassend:

Verwendung eines weiteren Bades
zum Ausglühen und Verlängern, wobei das Badmedium (75) flüssig oder heiße Luft ist; Aufrechterhaltung des Badmediums (75) im sechsten Bad bei 40-80 Grad C; und
Durchführung der Fluoreszenzabbildung durch das sechste Bad bei konstanter Temperatur, wobei das Badmedium und mindestens ein Teil des sechsten Bades transparent ist, um die Übertragung von Beleuchtungslicht von einer Lichtquelle und die Übertragung von emittiertem Licht von dem (den) Reaktor(en) (15) zu ermöglichen.

## Revendications

1. Appareil de traitement thermique de l'acide nucléique dans un profil thermique, l'appareil utilisant des milieux de bain (75) et un support de réacteur (33) pour maintenir le(s) réacteur(s) (15) chacun accommodant le matériau de réaction (21) contenant l'acide nucléique et le(s) réacteur(s) (15) étant sous une forme quelconque telle que tube(s) ou plaque(s) à puits ou puce(s) ou cartouche(s),
l'appareil comprenant:

un premier bain (50) ;

un second bain (51),

les milieux de bain (75) sont fournis respectivement dans le premier bain (50) et le second bain (51), dans lequel les milieux de bain (75) dans le premier bain (50) et le second bain (51) respectivement peuvent être maintenus à deux températures différentes, $T_{HAUTE}$ et $T_{BASSE}$; et

un moyen de transfert (85) configuré pour faire passer le(s) réacteur(s) (15) d'être dans les deux bains (50, 51) dans une pluralité de cycles thermiques à atteindre alternativement :

- une température cible haute prédéterminée $T_{HT}$, et
- une température cible basse prédéterminée $T_{LT}$,

tandis que l'appareil est configuré pour s'adapter à une caractéristique de décalage de température définie par au moins une condition du groupe constitué :

a) la $T_{HT}$ est inférieure à la $T_{HAUTE}$, et
b) la $T_{LT}$ est supérieure à la $T_{BASSE}$,

les moyens de transfert (85) étant utilisables par au moins un mode du groupe constitué :

un moyen de contrôle de mouvement guidé par la température (TeGMCM) qui est utilisable sur la base de la température en temps réel telle que détectée par un capteur de température du réacteur (38) pendant le cycle thermique,

et

un moyen de contrôle de mouvement guidé par le temps (TiGMCM) qui est utilisable sur la base des périodes de temps pendant lesquelles les(s) réacteur(s) (15) sont autorisés à être dans les bains (50, 51), le milieu de bain (75) dans l'un quelconque des bains (50, 51) étant dans n'importe quelle phase, y compris l'air, le liquide, le solide, la poudre et un mélange de l'un quelconque de ces ;

**caractérisé en ce que** le moyen de transfert (85) est configuré pour initier le décollage du (des) réacteur(s) (15) du (des) bain(s) (50, 51) lorsque le(s) réacteur(s) (15) atteint une première température de décollage qui est inférieure à la $T_{HT}$ et une seconde température de décollage supérieure à la $T_{LT}$ pour compenser les retards électromécaniques de fonctionnement qui provoquent involontairement un surchauffement ou un refroidissement excessif du (des) réacteur(s) (15).

2. Appareil selon la revendication 1, dans lequel le TiGMCM peut être calibré par l'utilisateur pour les périodes de temps.

3. Appareil selon la revendication 1, dans lequel le milieu de bain (75) dans le premier bain (50) est maintenu au-dessus de 100 degrés Celsius.

4. Appareil selon la revendication 1, dans lequel le milieu de bain (75) dans le second bain (51) est maintenu en dessous de la température ambiante.

5. Appareil selon la revendication 1, dans lequel un premier décalage entre la $T_{HAUTE}$ et la $T_{HT}$ se situe dans une plage de 1 - 400 degrés Celsius et un second décalage entre la $T_{BASSE}$ et la $T_{LT}$ se situe dans une plage de 1 - 100 degrés Celsius.

6. Appareil selon la revendication 1, en outre comprenant le(s) couvercle(s) de bain sur le(s) bain(s) (50, 51), le(s) couvercle(s) s'ouvrant pour permettre au(x) réacteur(s) (15) d'être dans le(s) bain(s) (50, 51) et se fermant après que le(s) réacteur(s) (15) a/ont été retiré(s) du (des) bain(s) (50, 51).

7. Procédé de traitement thermique de l'acide nucléique dans un profil thermique, le procédé comprenant:

utiliser un support de réacteur (33) pour maintenir le(s) réacteur(s) (15) chacun accommodant le matériau de réaction (21) contenant l'acide nucléique et le(s) réacteur(s) (15) étant sous une forme quelconque telle que tube(s) ou plaque(s) à puits ou puce(s) ou cartouche(s);

utiliser un appareil comprenant un premier bain (50) et un second bain (51);

maintenir les milieux de bain (75) dans le premier bain (50) à la température $T_{HAUTE}$ et dans le second bain (51) à la température $T_{BASSE}$; et

utiliser un moyen de transfert (85) dans l'appareil pour permettre au(x) réacteur(s) (15) d'être dans les deux bains (50, 51) dans une pluralité de cycles thermiques pour atteindre alternativement :

- une température cible haute prédéterminée $T_{HT}$, et
- une température cible basse prédéterminée $T_{LT}$, et

adapter à une caractéristique de décalage de température définie par au moins une condition du groupe constitué :

a. la $T_{HT}$ est inférieure à la $T_{HAUTE}$, et
b. la $T_{LT}$ est supérieure à la $T_{BASSE}$, et

les moyens de transfert (85) étant utilisables par au moins un mode du groupe constitué :

un moyen de contrôle de mouvement guidé par la température (TeGMCM) qui est utilisable sur la base de la température en temps réel telle que détectée par un capteur de température du réacteur (38) pendant le cycle thermique, et
un moyen de contrôle de mouvement guidé par le temps (TiGMCM) qui est utilisable sur la base des temps pendant lesquelles les(s) réacteur(s) (15) sont autorisés à être dans les bains (50, 51).
le milieu de bain (75) dans l'un quelconque des bains (50, 51) étant dans n'importe quelle phase, y compris l'air, le liquide, le solide, la poudre et un mélange de l'un quelconque de ces ;
**caractérisé en ce que** le procédé comprend en outre le calibrage du moyen de transfert (85) pour initier le décollage du (des) réacteur(s) (15) du (des) bain(s) (50, 51) lorsque le(s) réacteur(s) (15) atteint une première température de décollage qui est inférieure à la $T_{HT}$ et une seconde température de décollage supérieure à la $T_{LT}$ pour compenser les retards électromécaniques de fonctionnement qui provoquent involontairement un surchauffement ou un refroidissement excessif du (des) réacteur(s) (15).

8. Procédé selon la revendication 7, comprenant en outre :
maintenir le milieu de bain (75) dans le premier bain (50) au-dessus de 100 degrés Celsius.

9. Procédé selon la revendication 7, comprenant en outre :
maintenir le milieu de bain (75) dans le second bain (51) en dessous de la température ambiante.

10. Procédé selon la revendication 7, comprenant en outre :

régler un premier décalage entre la $T_{HAUTE}$ et la $T_{HT}$ se situe dans la plage de 1 - 400 degrés Celsius ; et
régler un second décalage entre la $T_{BASSE}$ et la $T_{LT}$ se situe dans la plage de 1 - 100 degrés Celsius.

11. Procédé selon la revendication 7, comprenant en outre :
ajouter un second liquide à un premier liquide dans le milieu du bain (75) dans le premier bain (50), le point d'ébullition du second liquide étant supérieur à celui du premier liquide.

12. Procédé selon la revendication 11, dans lequel le point d'ébullition d'un mélange du premier liquide et du second liquide est supérieur à 100 °C.

13. Appareil selon la revendication 1, dans lequel un premier décalage entre la $T_{HAUTE}$ et la $T_{HT}$ se situe dans une plage de 10 - 400 degrés Celsius et un second décalage entre la $T_{BASSE}$ et la $T_{LT}$ se situe dans une plage de 10 - 100 degrés Celsius.

14. Appareil selon la revendication 1, en outre comprenant: un autre bain dans lequel le milieu du bain (75) est un liquide ou de l'air chaud qui peut être maintenu à 40 - 80 degrés Celsius, dans lequel le milieu du bain (75) et au moins une portion du sixième bain est transparent pour permettre la transmission de la lumière d'éclairage d'une source de lumière et la transmission de la lumière émise par le(s) réacteur(s) (15).

15. Procédé selon la revendication 8, comprenant en outre :

l'utilisation d'un autre bain pour le recuit et l'extension, dans lequel le milieu du bain (75) est liquide ou de l'air chaud ;
maintenir le milieu de bain (75) dans le sixième bain à 40 - 80 degrés C; et

conduisant l'imagerie par fluorescence à travers le sixième bain à température constante,
dans lequel le milieu du bain et au moins une portion du sixième bain est transparent pour permettre la transmission de la lumière d'éclairage d'une source de lumière et la transmission de la lumière émise par le(s) réacteur(s) (15).

FIG.1a

FIG.1b

FIG.1c

FIG.2

FIG 3

FIG 4a

FIG 4b

FIG 5a

FIG 5b

Fig 6(a)

Fig 6(b)

EP 3 463 669 B1

FIG 7(a)

FIG 7(b)

FIG 7(c)

EP 3 463 669 B1

FIG 7(d)

FIG 7(e)

29

FIG 8(a)

FIG 8(b)

FIG 8(c)

Fig 9 (a)

Fig 9 (b)

Fig 9 (c)

Fig 9 (d)

Fig. 10 (a)

Fig. 10 (b)

Fig 11 (a)

Fig 11 (b)

Fig 11 (c)

Fig 11 (d)

Fig 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013177429 A2

- WO 2010030647 A1

**Non-patent literature cited in the description**

- **Y.A. CENGEL ; A.J. GHAJAR.** Heat and Mass Transfer: Fundamentals and Applications. Mc-Graw-Hill Education, 2015